# EUROPEAN PATENT SPECIFICATION

(11) **EP 1 611 251 B1**
(45) Date of publication and mention of the grant of the patent: **09.06.2010**
(21) Application number: 04721774.0
(22) Date of filing: 19.03.2004
(51) Int. Cl.: G01N 33/68

(54) **THERAPEUTIC, PROPHYLACTIC AND DIAGNOSTIC AGENTS**
THERAPEUTISCHE, PROPHYLAKTISCHE UND DIAGNOSTISCHE WIRKSTOFFE
AGENTS THERAPEUTIQUES, PROPHYLACTIQUES ET DIAGNOSTIQUES

(30) Priority: 21.03.2003 AU 2003901325
(43) Date of publication of application: 04.01.2006
(73) Proprietor: Murdoch Childrens Research Institute, Parkville, VIC 3052 (AU); Melbourne Health, Parkville, Victoria 3050 (AU)
(72) Inventor: VISVANATHAN, Kumar, Fitzroy, Victoria 3065 (AU); RIORDAN, Stephen, Kingsford, New South Wales 2032 (AU); LOCARNINI, Stephen, St Kilda East, Victoria 3183 (AU)
(74) Representative: Jones, Elizabeth Louise
(86) International application number: PCT/AU2004/000349
(87) International publication number: WO 2004/083455

(56) References cited:
- WO-A1-01/36488
- HUANG XIAO X ET AL: "An extended analysis of the liver transcriptome in hepatitis C virus associated cirrhosis" HEPATOLOGY, vol. 36, no. 4 Part 2, October 2002 (2002-10), page 439A, XP009073813 & 53RD ANNUAL MEETING ON THE LIVER; BOSTON, MA, USA; NOVEMBER 01-05, 2002 ISSN: 0270-9139
- AUSTIN ANDREW S ET AL: "Rapid upregulation of toll-like receptor (TLR) 4 and tumour necrosis factor alpha (TNF-alpha) expression in lipopolysaccharide (LPS) stimulated monocytes in whole blood in alcoholic cirrhosis" GASTROENTEROLOGY, vol. 122, no. 4 Suppl. 1, April 2002 (2002-04), pages A.648-A.649, XP009073818 & DIGESTIVE DISEASE WEEK AND THE 103RD ANNUAL MEETING OF THE AMERICAN GASTROENTEROLOGICAL ASSOCIATION; SAN FRANCISCO, CA, USA; MAY 19-22, 2002 ISSN: 0016-5085
- MANIGOLD ET AL.: 'Differential expression of toll-like receptors 2 and 4 in patients with liver cirrhosis' EUROPEAN JOURNAL OF GASTROENTEROLOGY & HEPATOLOGY vol. 15, no. 3, 2003, pages 275 - 282, XP009073723
- MANIGOLD ET AL.: 'Upregulation of toll-like receptor-2 in patients with liver cirrhosis' HEPATOLOGY vol. 30, no. 4, 1999, page 434A, XP009073728
- O'NEIL L.: 'The toll/interleukin-1 receptor domain: a molecular switch for inflammation and host defence' BIOCHEMICAL SOCIETY TRANSACTIONS vol. 28, no. 5, 2000, pages 557 - 563, XP002264057
- PAIK ET AL.: 'Toll-like receptor 4 mediates inflammatory signaling by bacterial lipopolysaccharide in human hepatic stellate cells' HEPATOLOGY vol. 37, no. 5, 2003, pages 1043 - 1055, XP008073073
- RIORDAN ET AL.: 'Peripheral blood mononuclear cell expression of toll-like receptors and relation to cytokine levels in cirrhosis' HEPATOLOGY vol. 37, no. 5, 2003, pages 1154 - 1164, XP008073072
- RIORDAN ET AL.: 'Toll-like receptor expression in cirrhosis' GUT vol. 52, no. SUPPL.1, 2003, page A2, XP008073075
- VISVANATHAN ET AL.: 'Up-regulation of toll-like receptor expression in chronic hepatitis C: correlation with circulating pro-inflammatory cytokine levels and hepatic necro-inflammatory activity' GUT vol. 52, no. SUPPL.1, 2003, page A35, XP009073796
- VISVANATHAN ET AL.: 'Impaired toll-like receptor expression in chronic hepatitis B' GUT vol. 52, no. SUPPL.1, 2003, page A36, XP009073794

## Description

### BACKGROUND OF THE INVENTION

### FIELD OF THE INVENTION

The present invention provides compounds for use in the treatment and prophylaxis of infection in mammals and avian species by Hepatitis B or Hepatitis C virus (HBV and HCV). In particular, the present invention enables a determination of whether, including a prediction of the level of likelihood that, a subject will respond to therapeutic or prophylactic intervention of HBV or HCV infection.

### DESCRIPTION OF THE PRIOR ART

Bibliographic details of the publications referred to in this specification are also collected at the end of the description.

Reference to any prior art in this specification is not, and should not be taken as, an acknowledgment or any form of suggestion that this prior art forms part of the common general knowledge in any country.

Over 170 million people are infected with the Hepatitis C virus (HCV) worldwide, resulting in a large disease burden and significant mortality. HCV is rarely cleared in the acute phase of the infection and most patients become chronically infected; a proportion of these patients develop progressive liver disease and fibrosis. The outcome of infection depends on the immune responses of both the innate and cognate immune systems, and these in turn are orchestrated by networks of cytokines and chemokines.

Hepatitis B virus (HBV) also causes debilitating disease conditions and can lead to acute liver failure. HBV is a DNA virus which replicates *via* an RNA intermediate and utilizes reverse transcription in its replication strategy. The HBV genome is of a complex nature having a partially double-stranded DNA structure with overlapping open reading frames encoding surface, core, polymerase and X genes.

The host virus relationship is a dynamic process in which many viruses such as HCV and HBV attempt to maximize their visibility while the host attempts to prevent and eradicate infection. Inititally, a virus must bind and enter a target cell and migrate to the appropriate cellular compartment in order to replicate and infect other cells. Infected cells may be triggered by the virus to produce cyokines (e.g. TNF-α and IFN-γ) that inhibit one or more stages of the viral replication cycle, thereby limiting the extent of the infection.

Host monocytes and macrophages play a key role in the early response to the virus as they secrete pro-inflammatory cytokines, such as IL-1, TNF-α, IL-6, IL-12 and IL-18 that have indirect and direct effects on the infection. They can recruit further monocytes, natural killer (NK) cells and T-cells to perform functions and they can also help switch the to appropriate Th function to help eradicate the virus.

Innate immunity to microbial pathogens, leading to the production of these pro-inflammatory cytokines, occurs as a result of the activation of Toll Like Receptors (TLRs). The role of TLRs involving bacterial products, e.g. endotoxin and peptidoglycan has recently been clarified (Akashi et al., J Immunol. 164: 3471-3475, 2000; Takeuchi et al., Immunity, 11: 443-451, 1999; Tapping et al., J Immunol. 165: 5780-5787, 2000). More than 10 TLRs have been identified and they play an important role in activation by a number of different bacteria. Recently, this has been extended to viruses with the demonstration that respiratory syncytial virus (RSV) stimulates TLR-4 in a murine model (Kurt-Jones et al., Nat Immunol, 1: 398-401, 2000; Haeberle et al., J Infect Dis. 186: 1199-1206, 2002). In addition, Measles Virus (MV) has been shown to activate TLR-2 dependent signals (Bieback et al., J Virol, 76: 8729-8736, 2002) and double-stranded DNA (the core of many viruses) has been shown to directly mediate responses to through TLR-3 (Matsumoto et al., Biochem Biophys Res Commun. 293: 1364-1369, 2002).

Circulating levels of pro-inflammatory cytokines such as TNF-α are significantly increased in patients with cirrhosis and on-going liver injury (Khoruts et al., Hepatology 13: 267-276, 1991; Tilg et al., Gastroenterology 103: 264-274, 1992; Lee et al., Scand J Gastroenterol 31: 500-505, 1996; Genesca et al., Am J Gastroenterol. 94: 169-177, 1999; von Baehr et al., Gut 47: 281-287, 2000; Andus et al., Hepatology 13: 364-375, 1991; Enomoto et al., J Gastroenterol Hepatol. 15(Suppl): D20-D25, 2000; Neuman et al., J Gastroenterol Hepatol. 17: 196-202, 2002). TNF-α has been shown to be a critical factor in the development of alcohol-induced acute liver injury in animal models (Iimuro et al., Hepatology 26: 1530-1537, 1997; Yin et al., Gastroenterology 117: 942-952, 1999). Activation of macrophages by endotoxin, a component of the cell walls of Gram-negative bacteria, plays a key role in the pathogenesis of TNF-α over-production and liver injury in such models (Enomoto et al., J. Biomed Sci. 8: 20-27, 2001). Several factors promote endotoxaemia in this setting, including increased translocation of endotoxin from the gut lumen and a reduction in hepatic clearance capacity (Nanji et al., Am J Pathol. 142: 367-373, 1993; Rivera et al., Am J Physiol. 275: G1252-1258, 1998). In the clinical setting, several studies have shown significant, though relatively modest, increases in circulating endotoxin levels in patients with cirrhosis (Khoruts *et al.,* 1991, *supra;* von Baehr *et al.,* 2000, *supra*; Fukui et al., J Hepatol. 12: 162-169, 1991; Lin et al., J Hepatol. 22: 165-172, 1995; Chan et al., Scand J Gastroenterol. 32: 942-946, 1997; Hanck et al., Gut 49: 106-111, 2001) and endotoxaemia has been assumed to be responsible for the increased circulating TNF-α levels in this group (Tilg *et al.,* 1992, *supra*; von Baehr *et al*., 2000, *supra*; Schafer et al., Z Gastroenterol. 33: 503-508, 1995; Deviere et al., Gastroenterology 103: 1296-1301, 1992). Endotoxaemia has also been assumed to be responsible for the elevated peripheral blood levels of anti-inflammatory mediators such as soluble TNF receptors (sTNFRs) found in cirrhosis (von Baehr *et al*., 2000, *supra*; Tilg et al., Hepatology, 18: 1132-1138; 1993). Nonetheless, a significant correlation between circulating endotoxin and cytokine levels has generally not been demonstrated (Khoruts *et al*, 1991, *supra;* Tilg *et al*, 1993, *supra*; von Baehr *et al*, 2000, *supra*; Chan *et al*., 1997, *supra)* raising the possibility that, unlike in animal models, stimuli other than endotoxin may be important.

It has recently been demonstrated that TNF-α production by macrophages in response to microbial stimuli is critically dependent upon activation of TLRs (Medzhitov et al., N Engl J Med. 343: 338-344, 2000; Yoshimura et al., J Immunol. 163: 1-5, 1999; Akira et al., Nature Immunolog, 2: 675-680, 2001). As indicated above, TLRs play a critical role in the induction of innate immunity to microbial pathogens *via* recognition of conserved molecular patterns. In particular, it is known that TLR-4, in association with CD14, is responsible for signal transduction leading to TNF-α production in response to endotoxin. In contrast, TLR-2 is required for signaling in response to a number of Gram-positive microbial stimuli, including whole bacteria and cell wall components such as peptidoglycan and lipoteichoic acid (LTA) (Medzhitov *et al*., 2000, *supra*; Yoshimura *et al*., 1999; *supra;* Akira *et al*., 2001, *supra)*.

TLR-2 expression in peripheral blood mononuclear cells (PBMCs) has been shown to be increased in patients with liver cirrhosis (Marigold et al., Hepatology, 30: 434A, 1999), while TLR-4 expression has been reported to be down-regulated (Marigold et al., European Journal of Gastroenterology & Hepatology, 15: 275-282, 2003). This latter report also found intrahepatic expression of TLR-2 and TLR-4 to be unchanged in cirrhotic-liver patients. In a study using whole blood from alcoholic cirrhotics, enhanced sensitivity of circulating monocytes to TNF-α secreted in response to a low concentration of LPS was attributed to rapid up-regulation of TLR-4 cell surface expression in response to LPS (Austin et al., Gastroenterology, 122: A648-A649, 2002). TLR-2, TLR-4 and TLR-9 were amongst many genes found to be up-regulated in an analysis of the liver transcriptome of HCV-cirrhotic-liver patients (Huang et al., Hepatology, 36: 439A, 2002).

There is a need to investigate the role of TLRs in infection with pathogenic entities such as microorganisms or viruses or other diseased states.

### SUMMARY OF THE INVENTION

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

The present description identifies cell surface markers which are differentially affected in response to infection by a pathogenic organism or virus or in response to other disease conditions. The cell surface markers are, therefore, useful therapeutic and/or diagnostic targets. In particular, the present description identifies Toll-like receptors (TLRs) as being useful therapeutic and diagnostic markers for infection by pathogenic agents such as microorganisms and viruses or development of other disease conditions. In addition, the TLRs are useful indicators as to the potential responsiveness of a subject to therapeutic intervention including enabling a prediction as to the likelihood or otherwise of a subject responding favourably to therapeutic intervention. Such a prediction is a form of risk assessment. TLRs contain ectodomains with leucine-rich repeats and comprise intracellular motifs which are highly homologous to intracellular signaling domains of interleukin-1 receptor type I (IL-1RI) and IL-1RI accessory protein. Eleven TLRs have so far been identified designated TLR-1 through TLR-11.

It has been identified that TLRs, and in particular TLR-2 and TLR-4, are differentially affected on peripheral blood mononuclear cells (PBMCs) and in particular CD14+ PMBC (i.e. monocytes) and liver cells following infection by a pathogenic agent such as a microorganism or virus. TLRs and, in particular, TLR-2 and TLR-4 are, therefore, useful targets for therapeutic or prophylactic agents to treat or help prevent infection by a pathogenic agent. TLRs are also differentially affected in to other disease conditions such as but not limited to cirrhosis and hepatocellular carcinoma (HCC). They are also useful diagnostic targets to determine whether a subject is or has been infected by a pathogenic entity or whether the subject is predisposed to or has a persistent infection or has another disease condition and can be used as a clinical or epidemiological management tool.

The present invention provides, therefore, therapeutic and/or prophylactic agents capable of modulating levels of TLR-2 and/or TLR-4 for use in treating a subject infected with HCV or HBV. For example, during infection with Hepatitis C virus (HCV), TLR-2 and TLR-4 levels are elevated in PBMCs and in particular monocytes and liver cells. Consequently, therapeutic and prophylactic agents may be designed or selected which down-regulate these TLRs. Conversely, infection with Hepatitis B virus (HBV) down-regulates TLR-2. Consequently, therapeutic agents may be designed or selected which up-regulate TLR-2 levels. In addition, TLR-2 levels are elevated in liver conditions such as cirrhosis or HCC.

The present description discloses methods for diagnosis or assessment of infection by a pathogenic agent such as a microorganism or virus by determining the levels of TLRs such as TLR-2 and/or TLR-4 on PBMCs and/or liver cells. In one example, the failure for TLR-2 and/or TLR-4 levels to alter (eg. increase or decrease) during early phase treatment provides an indication that the treatment protocol has some probability of not working.

The present invention provides, therefore, therapeutic and diagnostic agents for use in the treatment and/or prophylaxis of a subject infected with HCV or HBV. The subject may be a mammal or avian species.

The present invention further provides an *in vitro* diagnostic method for monitoring a response to treatment of a subject against HCV or HBV infection as well as for determining whether a subject will respond to an anti-HCV treatment or an anti-HBV treatment or for predicting the outcome of a therapeutic protocol directed against infection by HCV or HBV.

Preferred mammals are humans. Preferred avian species are poultry birds.

### BRIEF DESCRIPTION OF THE FIGURES

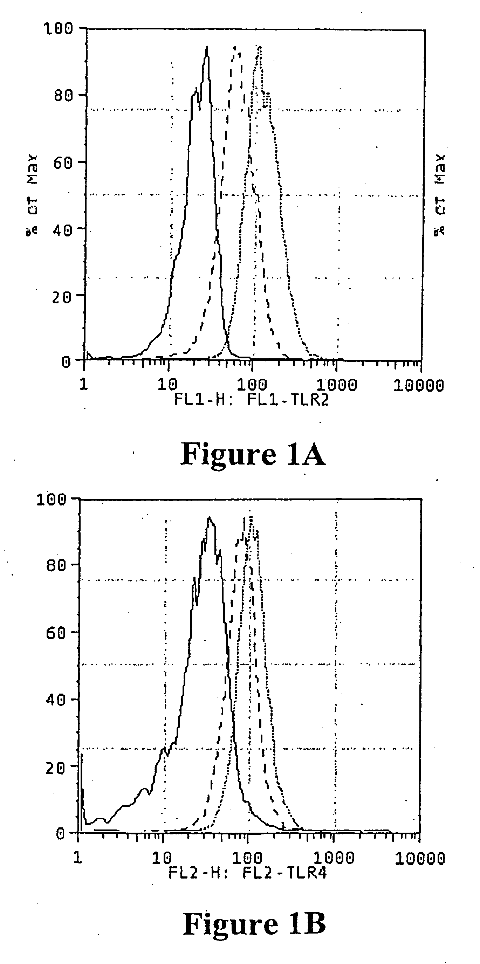

**Figures 1A and 1B** are graphical representations showing TLR profiles in control subjects and patients with CHC. Whole blood was stained with directly conjugated antibodies to CD14 and either TLR-2 or TLR-4. Diagrams represent 10,000 CD14-gated cells. The solid line represents expression of the isotype control, the dotted line the control subject and the dashed line the CHC patient.

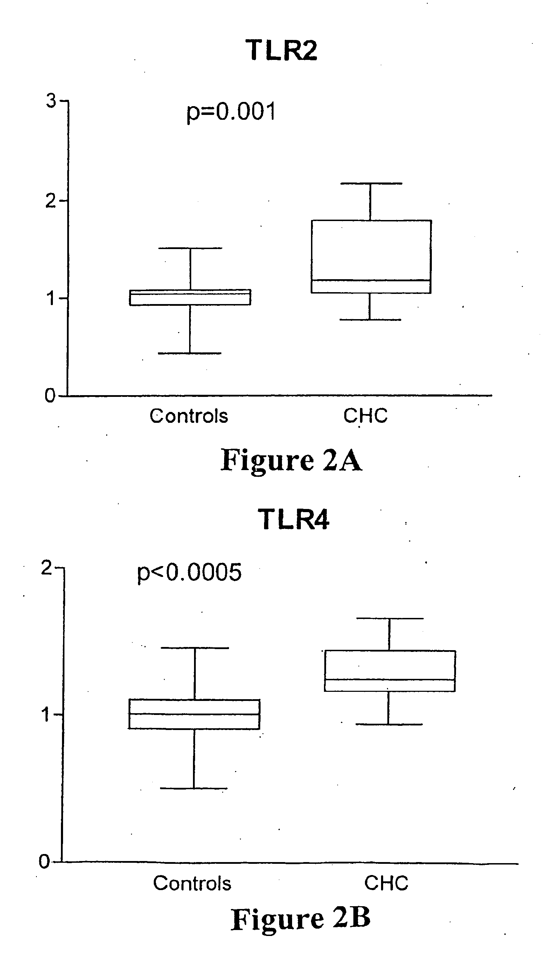

**Figures 2A and 2B** are graphical representations showing TLR-4 and TLR-2 expression on CD14^{+ve} peripheral blood monocytes in control subjects and patients with HCV infection. TLR-2 and TLR-4 expression was significantly increased in patients with HCV infection.
**Figure 3** is a graphical representation of data from 6 patients showing *in vitro* expression in peripheral blood of TNF-α (Figure 3 a and b), TLR2 (Figure 3c and d) and TLR4 (Figure 3e and f) following stimulation with HBV. In which each of b,d and f shows a graphical representation of the average stimulation.
**Figure 4** is a graphical representation showing *in vitro* expression of TLR2 and TLR 4 in HepG2 cells following transduction with recombinant HBV/ baculovirus.

### DETAILED DESCRIPTION OF THE PREFERRED EMBODIMENT

The present invention is predicated in part on the determination that levels of particular cell surface markers correlate with infection by particular pathogenic entities such as microorganisms or viruses. More particularly, the present description discloses that TLR genes are differentially expressed during infection by pathogenic entities such as microorganisms or viruses.

It has been observed that infection by HCV results in elevated levels of TLR-2 and TLR-4 in CD14+ PBMCs (i.e. monocytes) and liver cells. The elevated levels of TLR-2 and TLR-4 may be used as a diagnostic indicator of HCV infection or a predisposition to or persistence of HCV infection. The level of the TLRs are also useful indications of the likelihood that a subject will respond favourably to the therapeutic intervention. Reference to "likelihood" includes making a prediction and making risk assessment of the likelihood or otherwise of success of the treatment protocol. Additionally, TLR-2 and TLR-4 become therapeutic targets for agents which down-regulate TLR-2 and/or TLR-4 levels.

It has further been observed that infection by HBV results in down-regulation of TLR-2 in CD14+ PBMCs and liver cells. Again, this enables the level of TLR-2 to be used as a diagnostic marker for HBV infection and as a target for agents to up-regulate TLR-2 in subjects infected by HBV or who have a predisposition to or persistence of infection with HBV. Normalization of levels of TLR-2 and/or TLR-4 is a prediction that a treatment protocol is working. Comparisons are conveniently made to pre-treatment levels or a database of normalized controls.

The present invention provides, therefore, agents which modulate levels of TLR-2 and/or TLR-4 for use in treating a subject infected with HCV or HBV, and *in vitro* diagnostic methods involving determining the levels of TLR-2 and/or TLR-4. Monitoring a therapeutic regimen and determining a subject's predisposition to or persistence of infection by a pathogenic entity, is contemplated by the present disclosure.

The present invention further provides an *in vitro* diagnostic method for monitoring a response to treatment of a subject against HCV or HBV infection as well as for determining whether a subject will respond to an anti-HCV treatment or an anti-HBV treatment or for predicting the outcome of a therapeutic protocol directed against infection by HCV or HBV. The present invention may be useful as a clinical or epidemiological management tool for HBV and/or HCV infection in animals such as mammals and in particular humans.

The present description discloses a method for detecting the presence of infection by a pathogenic agent or a predisposition thereto, said method comprising determining the level of TLR-2 and/or TLR-4 or a homolog thereof wherein an elevated or reduced level of TLR-2 and/or TLR-4 or a homolog thereof is indicative of infection by the pathogenic agent or predisposition thereto.

In one aspect, the invention provides an *in vitro* diagnostic method for monitoring a response to treatment of a subject against HCV or HBV infection said method comprising determining the level of TLR-2 and/or TLR-4 wherein the efficacy of the anti-HBV treatment is determined by an elevation in the level of TLR-2 and the efficacy of the anti-HCV treatment is determined by a reduction in the level of TLR-2 and TLR-4 compared to pre-treatment levels and/or standardized controls.

Another aspect provides an *in vitro* diagnostic method for determining whether a subject will respond to an anti-HCV treatment or an anti-HBV treatment said method comprising determining the level of TLR-2 and/or TLR-4 wherein the potential efficacy of the treatment is determined by an elevated level of TLR-2 and TLR-4 in a subject with HCV and a reduced level of TLR-2 in a subject with HBV.

Still yet a further aspect provides an *in vitro* diagnostic method for predicting the outcome of a therapeutic protocol directed against infection by HCV or HBV said method comprising determining the level of TLR-2 and/or TLR-4 wherein the efficacy of the therapeutic protocol is determined by an elevation in the level of TLR-2 during anti-HBV treatment and by a reduction in the level of TLR-2 and TLR-4 during anti-HCV treatment compared to pre-treatment levels and/or standardized controls.

In accordance with the risk assessment aspects disclosed herein, one would expect change in early phase treatment to result in a change or a trend to change levels of TLR-2 and/or TLR-4. When that occurs, the likelihood of successful therapeutic intervention is considered reasonable to high. If there is no trend to alter the TLR levels then this is an indication of a less than successful therapeutic protocol.

It is to be understood that unless otherwise indicated, the subject invention is not limited to specific formulations of components, manufacturing methods, dosage regimens, or the like, as such may vary. It is also to be understood that the terminology used herein is for the purpose of describing particular embodiments only and is not intended to be limiting.

It must be noted that, as used in the subject specification, the singular forms "a", "an" and "the" include plural aspects unless the context clearly dictates otherwise. Thus, for example, reference to a "compound" includes a single compound, as well as two or more compounds; reference to "an active agent" includes a single active agent, as well as two or more active agents; and so forth.

In describing and claiming the present invention and other aspects disclosed herein, the following terminology are used in accordance with the definitions set forth below.

The terms "compound", "active agent", "pharmacologically active agent", "medicament", "active" and "drug" are used interchangeably herein to refer to a chemical compound that induces a desired pharmacological and/or physiological effect. The terms also encompass pharmaceutically acceptable and pharmacologically active ingredients of those active agents specifically mentioned herein including but not limited to salts, esters, amides, prodrugs, active metabolites, analogs and the like. When the terms "compound", "active agent", "pharmacologically active agent", "medicament", "active" and "drug" are used, then it is to be understood that this includes the active agent *per se* as well as pharmaceutically acceptable, pharmacologically active salts, esters, amides, prodrugs, metabolites, analogs, etc. The term "compound" is not to be construed as a chemical compound only but extends to peptides, polypeptides and proteins as well as genetic molecules such as RNA, DNA and chemical analogs thereof. Reference to a "peptide", "polypeptide" or "protein" includes molecules with a polysaccharide or lipopolysaccharide component. The term "potentiator" is an example of a compound, active agent, pharmacologically active agent, medicament, active and drug which up-regulates the level of a TLR such as TLR-2 or TLR-4. The term "up-regulates" encompasses increasing expression of a TLR gene as well as manipulating a component of the downstream signaling pathway. The term "antagonist" is an example of a compound, active agent, pharmacologically active agent, medicament, active and drug which down-regulates the level of a TLR, such as TLR-2 or TLR-4. Down-regulation may also involve elevating levels of an inhibitor of the TLR signaling pathway.

Accordingly, reference to a TLR also includes reference to the signaling pathway associated with interaction between a ligand and a TLR.

The present description contemplates, therefore, compounds useful in up-regulating a TLR such as TLR-2 and/or TLR-4 or general or specific TLR signaling. The terms "modulating" or its derivatives, such as "modulate" or "modulation", are used to describe up- or down-regulation. The compounds have an effect on reducing or preventing or treating infection by a pathogenic organism or virus. Examples of cells which carry the TLRs to be modulated are PBMCs and liver cells. A PBMC includes a CD14+ PMBC and in particular a monocyte and a liver cell includes a hepatocyte. Reference to a "compound", "active agent", "pharmacologically active agent", "medicament", "active" and "drug" includes combinations of two or more actives such as a potentiator or antagonist of TLR or TLR signaling. A "combination" also includes multi-part such as a two-part pharmaceutical composition where the agents are provided separately and given or dispensed separately or admixed together prior to dispensation.

For example, a multi-part pharmaceutical pack may have a modulator of a TLR and one or more anti-microbial or anti-viral agents.

The terms "effective amount" and "therapeutically effective amount" of an agent as used herein mean a sufficient amount of the agent to provide the desired therapeutic or physiological effect. Furthermore, an "effective TLR-potentiating amount" or an "effective TLR-antagonizing amount" of an agent is a sufficient amount of the agent to directly or indirectly up-regulate or down-regulate the function of a specific TLR such a TLR-2 or TLR-4 or to disrupt or potentiate TLR signaling. This may be accomplished by the agents acting as an agonist or antagonist of the TLR or its signaling components, by the agents which are or mimic components of the TLR signaling pathway, by agents which induce the TLR signaling pathway *via* other cellular receptors or by the agents antagonizing inhibitors of TLR signaling componets. Undesirable effects, e.g. side effects, are sometimes manifested along with the desired therapeutic effect; hence, a practitioner balances the potential benefits against the potential risks in determining what is an appropriate "effective amount". The exact amount required will vary from subject to subject, depending on the species, age and general condition of the subject, mode of administration and the like. Thus, it may not be possible to specify an exact "effective amount". However, an appropriate "effective amount" in any individual case may be determined by one of ordinary skill in the art using only routine experimentation.

By "pharmaceutically acceptable" carrier, excipient or diluent is meant a pharmaceutical vehicle comprised of a material that is not biologically or otherwise undesirable, i.e. the material may be administered to a subject along with the selected active agent without causing any or a substantial adverse reaction. Carriers may include excipients and other additives such as diluents, detergents, coloring agents, wetting or emulsifying agents, pH buffering agents, preservatives, and the like. A pharmaceutical composition may also be described depending on the formulation as a vaccine composition.

Similarly, a "pharmacologically acceptable" salt, ester, emide, prodrug or derivative of a compound as provided herein is a salt, ester, amide, prodrug or derivative that this not biologically or otherwise undesirable.

The terms "treating" and "treatment" as used herein refer to reduction in severity and/or frequency of symptoms of infection or disease, elimination of symptoms and/or underlying cause, prevention of the occurrence of symptoms of infection and/or their underlying cause and improvement or remediation of damage. Collateral damage, for example, following viral infection may be liver damage such as cirrhosis or HCC of the liver.

"Treating" a patient may involve prevention of infection or other disease condition or adverse physiological event in a susceptible individual as well as treatment of a clinically symptomatic individual by inhibiting an infection or other disease condition or downstream condition such as liver damage or cancer. In the present invention, such a condition or disorder is infection by HBV or HCV. Thus, for example, as disclosed herein the method of "treating" a patient with an infection or with a propensity for one to develop encompasses both prevention of the infection as well as treating the infection once established.

According to the present invention the infection is by HBV or HCV. Reference to "HBV" or "HCV" or their full terms such as "Hepatitis B virus" or "Hepatitis C virus" include all variants including variants resistant to particular therapeutic agents such as nucleoside analogs or immunological agents.

"Patient" as used herein refers to an animal, preferably a mammal and more preferably human who can benefit from the pharmaceutical formulations and methods disclosed herein. There is no limitation on the type of animal that could benefit from the presently described pharmaceutical formulations and methods. A patient regardless of whether a human or non-human animal may be referred to as an individual, subject, animal, host or recipient. The compounds and methods disclosed herein have applications in human medicine, veterinary medicine as well as in general, domestic or wild animal husbandry. For convenience, an "animal" includes an avian species such as a poultry bird, an aviary bird or game bird. A poultry bird such as a duck is a preferred example of an avian species.

The compounds (agents) contemplated by the present description may be large or small molecules, nucleic acid molecules (including antisense or sense molecules), peptides, polypeptides or proteins or hybrid molecules such as RNAi- or siRNA-complexes, ribozymes or DNAzymes. The compounds may need to be modified so as to facilitate entry into a cell. This is not a requirement if the compound interacts with an extracellular receptor. Examples of such agents include chemical agents and antibodies which interact with the TLR or genetic molecules which down-regulate or up-regulate expression of a gene encoding a TLR or compound of a TLR signaling pathway.

Preferred agents for use in the invention are selected from an antibody specific for TLR-2 or TLR-4 or an antigen-binding fragment thereof, a sense or antisense nucleic acid molecule to TLR-2 or TLR-4 and RNAi, siRNA, a ribozyme or a DNAzyme to TLR-2 or TLR-4.

As indicated above, the preferred animals are humans or other primates, livestock animals, laboratory test animals, companion animals or captive wild animals, as well as avian species.

Examples of laboratory test animals include mice, rats, rabbits, guinea pigs and hamsters. Rabbits and rodent animals, such as rats and mice, provide a convenient test system or animal model. Livestock animals include sheep, cows, pigs, goats, horses and donkeys. Non-mammalian animals such as avian species (such as ducks), zebrafish, amphibians (including cane toads) and *Drosophila* species such as *Drosophila melanogaster* are also contemplated.

The present description discloses, therefore, agents which antagonize or agonize (ie. potentiate or activate) TLRs such as TLR-2 and/or TLR-4.

The present description discloses methods of screening for such agents comprising, for example, contacting a candidate drug with a TLR such as TLR-2 or TLR-4 or a part thereof. Such a TLR molecule is referred to herein as a "target" or "target molecule". The screening procedure includes assaying (i) for the presence of a complex between the drug and the target, or (ii) an alteration in the expression levels of nucleic acid molecules encoding the target. One form of assay involves competitive binding assays. In such competitive binding assays, the target is typically labeled. Free target is separated from any putative complex and the amount of free (i.e. uncomplexed) label is a measure of the binding of the agent being tested to target molecule. One may also measure the amount of bound, rather than free, target. It is also possible to label the compound rather than the target and to measure the amount of compound binding to target in the presence and in the absence of the drug being tested. Such compounds may inhibit the target which is useful, for example, in finding inhibitors of TLR-2 and/or TLR-4 required for treating HCV infection or other disease condition or may protect TLR-2 or other components from being inhibited which would be required for treating HBV infection.

Another technique for drug screening provides high throughput screening for compounds having suitable binding affinity to a target and is described in detail in Geysen (International Patent Publication No. WO 84/03564). Briefly stated, large numbers of different small peptide test compounds are synthesized on a solid substrate, such as plastic pins or some other surface. The peptide test compounds are reacted with a target and washed. Bound target molecule is then detected by methods well known in the art. This method may be adapted for screening for non-peptide, chemical entities. This method, therefore, extends to combinatorial approaches to screening for target antagonists or agonists of TLRs such as TLR-2 or TLR-4.

Purified target can be coated directly onto plates for use in the aforementioned drug screening techniques. However, non-neutralizing antibodies to the target may also be used to immobilize the target on the solid phase. Antibodies specific for a TLR, such as TLR-2 or TLR-4, may also be useful as inhibitors of these TLRs such as in the treatment of HCV infection or other disease condition.

The present description also discloses the use of competitive drug screening assays in which neutralizing antibodies capable of specifically binding the target compete with a test compound for binding to the target or fragments thereof. In this manner, the antibodies can be used to detect the presence of any peptide which shares one or more antigenic determinants of the target.

Antibodies to a TLR, specifically TLR-2 or TLR-4 provided for by the invention, may be polyclonal or monoclonal although monoclonal antibodies are preferred. Antibodies may be prepared by any of a number of means. For the detection of a TLR, antibodies are generally but not necessarily derived from non-human animals such as primates, livestock animals (e.g. sheep, cows, pigs, goats, horses), laboratory test animals (e.g. mice, rats, guinea pigs, rabbits) and companion animals (e.g. dogs, cats). Generally, antibody based assays are conducted *in vitro* on cell or tissue biopsies. However, if an antibody is suitably deimmunized or, in the case of human use, humanized, then the antibody can be labeled with, for example, a nuclear tag, administered to a subject and the site of nuclear label accumulation determined by radiological techniques. The TLR antibody is regarded, therefore, as a pathogenic marker targeting agent. Accordingly, deimmunized forms of the antibodies may be used in pathogenic target imaging in human and non-human subjects. This is described further below.

For the generation of antibodies to TLR, the enzyme is required to be extracted from a biological sample whether this be from animal including human tissue or from cell culture if produced by recombinant means. Generally, monocytes and hepatocytes are a convenient source. The TLR can be separated from the biological sample by any suitable means. For example, the separation may take advantage of any one or more of TLR's surface charge properties, size, density, biological activity and its affinity for another entity (e.g. another protein or chemical compound to which it binds or otherwise associates). Thus, for example, separation of TLR from the biological sample may be achieved by any one or more of ultra-centrifugation, ion-exchange chromatography (e.g. anion exchange chromatography, cation exchange chromatography), electrophoresis (e.g. polyacrylamide gel electrophoresis, isoelectric focussing), size separation (e.g., gel filtration, ultrafiltration) and affinity-mediated separation (e.g. immunoaffinity separation including, but not limited to, magnetic bead separation such as Dynabead (trademark) separation, immunochromatography, immuno-precipitation). Choice of the separation technique(s) employed may depend on the biological activity or physical properties of the particular TLR sought or from which tissues it is obtained.

Preferably, the separation of TLR from the biological fluid preserves conformational epitopes present on the kinase and, thus, suitably avoids techniques that cause denaturation of the enzyme. Persons of skill in the art will recognize the importance of maintaining or mimicking as close as possible physiological conditions peculiar to the TLR (e.g. the biological sample from which it is obtained) to ensure that the antigenic determinants or active site/s on the TLR, which are exposed to the animal, are structurally identical to that of the native enzyme. This ensures the raising of appropriate antibodies in the immunized animal that would recognize the native enzyme.

Immunization and subsequent production of monoclonal antibodies can be carried out using standard protocols as for example described by Köhler and Milstein (Kohler and Milstein, Nature 256: 495-499, 1975; Kohler and Milstein, Eur. J. Immunol. 6(7): 511-519, 1976), Coligan et al. ("Current Protocols in Immunology, John Wiley & Sons, Inc., 1991-1997) or Toyama et al. (Monoclonal Antibody, Experiment Manual", published by Kodansha Scientific, 1987). Essentially, an animal is immunized with a TLR or a sample comprising a TLR by standard methods to produce antibody-producing cells, particularly antibody-producing somatic cells (e.g. B lymphocytes). These cells can then be removed from the immunized animal for immortalization.

Where a fragment of TLR is used to generate antibodies, it may need to first be associated with a carrier. By "carrier" is meant any substance of typically high molecular weight to which a non- or poorly immunogenic substance (e.g. a hapten) is naturally or artificially linked to enhance its immunogenicity.

Immortalization of antibody-producing cells may be carried out using methods which are well-known in the art. For example, the immortalization may be achieved by the transformation method using Epstein-Barr virus (EBV) (Kozbor et al., Methods in Enzymology 121: 140, 1986). Preferably, antibody-producing cells are immortalized using the cell fusion method (described in Coligan *et al*., 1991-1997, *supra*), which is widely employed for the production of monoclonal antibodies. In this method, somatic antibody-producing cells with the potential to produce antibodies, particularly B cells, are fused with a myeloma cell line. These somatic cells may be derived from the lymph nodes, spleens and peripheral blood of primed animals, preferably rodent animals such as mice and rats. Mice spleen cells are particularly useful. It would be possible, however, to use rat, rabbit, sheep or goat cells, or cells from other animal species instead.

Specialized myeloma cell lines have been developed from lymphocytic tumours for use in hybridoma-producing fusion procedures (Kohler and Milstein, 1976, *supra*; Shulman et al., Nature 276: 269-270, 1978; Volk et al., J. Virol. 42(1): 220-227, 1982). These cell lines have been developed for at least three reasons. The first is to facilitate the selection of fused hybridomas from unfused and similarly indefinitely self-propagating myeloma cells. Usually, this is accomplished by using myelomas with enzyme deficiencies that render them incapable of growing in certain selective media that support the growth of hybridomas. The second reason arises from the inherent ability of lymphocytic tumour cells to produce their own antibodies. To eliminate the production of tumour cell antibodies by the hybridomas, myeloma cell lines incapable of producing endogenous light or heavy immunoglobulin chains are used. A third reason for selection of these cell lines is for their suitability and efficiency for fusion.

Many myeloma cell lines may be used for the production of fused cell hybrids, including, e.g. P3X63-Ag8, P3X63-AG8.653, P3/NS1-Ag4-1 (NS-1), Sp2/0-Ag14 and S194/5.XXO.Bu.1. The P3X63-Ag8 and NS-1 cell lines have been described by Köhler and Milstein (1976, *supra*). Shulman *et al*. (1978, *supra*) developed the Sp2/0-Ag14 myeloma line. The S194/5.XXO.Bu.1 line was reported by Trowbridge (J. Exp. Med. 148(1): 313-323, 1978).

Methods for generating hybrids of antibody-producing spleen or lymph node cells and myeloma cells usually involve mixing somatic cells with myeloma cells in a 10:1 proportion (although the proportion may vary from about 20:1 to about 1:1), respectively, in the presence of an agent or agents (chemical, viral or electrical) that promotes the fusion of cell membranes. Fusion methods have been described (Kohler and Milstein, 1975, *supra*; Kohler and Milstein, 1976, *supra*; Gefter et al., Somatic Cell Genet. 3: 231-236, 1977; Volk *et al*., 1982, *supra*). The fusion-promoting agents used by those investigators were Sendai virus and polyethylene glycol (PEG).

Because fusion procedures produce viable hybrids at very low frequency (e.g. when spleens are used as a source of somatic cells, only one hybrid is obtained for roughly every 1x10⁵ spleen cells), it is preferable to have a means of selecting the fused cell hybrids from the remaining unfused cells, particularly the unfused myeloma cells. A means of detecting the desired antibody-producing hybridomas among other resulting fused cell hybrids is also necessary. Generally, the selection of fused cell hybrids is accomplished by culturing the cells in media that support the growth of hybridomas but prevent the growth of the unfused myeloma cells, which normally would go on dividing indefinitely. The somatic cells used in the fusion do not maintain long-term viability in *in vitro* culture and hence do not pose a problem. In the example of the present invention, myeloma cells lacking hypoxanthine phosphoribosyl transferase (HPRT-negative) were used. Selection against these cells is made in hypoxanthine/aminopterin/thymidine (HAT) medium, a medium in which the fused cell hybrids survive due to the HPRT-positive genotype of the spleen cells. The use of myeloma cells with different genetic deficiencies (drug sensitivities, etc.) that can be selected against in media supporting the growth of genotypically competent hybrids is also possible.

Several weeks are required to selectively culture the fused cell hybrids. Early in this time period, it is necessary to identify those hybrids which produce the desired antibody, so that they may subsequently be cloned and propagated. Generally, around 10% of the hybrids obtained produce the desired antibody, although a range of from about 1 to about 30% is not uncommon. The detection of antibody-producing hybrids can be achieved by any one of several standard assay methods, including enzyme-linked immunoassay and radioimmunoassay techniques as, for example, described in Kennet et al. (Monoclonal Antibodies and Hybridomas: A New Dimension in Biological Analyses, pp 376-384, Plenum Press, New York, 1980) and by FACS analysis (O'Reilly et al., Biotechniques 25: 824-830, 1998).

Once the desired fused cell hybrids have been selected and cloned into individual antibody-producing cell lines, each cell line may be propagated in either of two standard ways. A suspension of the hybridoma cells can be injected into a histocompatible animal. The injected animal will then develop tumours that secrete the specific monoclonal antibody produced by the fused cell hybrid. The body fluids of the animal, such as serum or ascites fluid, can be tapped to provide monoclonal antibodies in high concentration. Alternatively, the individual cell lines may be propagated *in vitro* in laboratory culture vessels. The culture medium containing high concentrations of a single specific monoclonal antibody can be harvested by decantation, filtration or centrifugation, and subsequently purified.

The cell lines are tested for their specificity to detect the TLR of interest by any suitable immunodetection means. For example, cell lines can be aliquoted into a number of wells and incubated and the supernatant from each well is analyzed by enzyme-linked immunosorbent assay (ELISA), indirect fluorescent antibody technique, or the like. The cell line(s) producing a monoclonal antibody capable of recognizing the target TLR but which does not recognize non-target epitopes are identified and then directly cultured *in vitro* or injected into a histocompatible animal to form tumours and to produce, collect and purify the required antibodies.

These antibodies are TLR-specific. This means that the antibodies are capable of distinguishing a particular TLR from other molecules. More broad spectrum antibodies may be used provided that they do not cross-react with molecules in a normal cell.

Where the monoclonal antibody is destined for use as a therapeutic agent such as to inhibit TLR-2 or TLR-4, then, it will need to be deimmunized with respect to the host into which it will be introduced (e.g. a human). The deimmunization process may take any of a number of forms including the preparation of chimeric antibodies which have the same or similar specificity as the monoclonal antibodies prepared according to the present invention. Chimeric antibodies are antibodies whose light and heavy chain genes have been constructed, typically by genetic engineering, from immunoglobulin variable and constant region genes belonging to different species. Thus, once a hybridoma producing the desired monoclonal antibody is obtained, techniques are used to produce interspecific monoclonal antibodies wherein the binding region of one species is combined with a non-binding region of the antibody of another species (Liu et al., Proc. Natl. Acad. Sci. USA 84: 3439-3443, 1987). For example, complementary determining regions (CDRs) from a non-human (e.g. murine) monoclonal antibody can be grafted onto a human antibody, thereby "humanizing" the murine antibody (European Patent No. 0 239 400; Jones et al., Nature 321: 522-525, 1986; Verhoeyen et al., Science 239: 1534-1536, 1988; Richmann et al., Nature 332: 323-327, 1988). In this case, the deimmunizing process is specific for humans. More particularly, the CDRs can be grafted onto a human antibody variable region with or without human constant regions. The non-human antibody providing the CDRs is typically referred to as the "donor" and the human antibody providing the framework is typically referred to as the "acceptor". Constant regions need not be present, but if they are, they must be substantially identical to human immunoglobulin constant regions, i.e. at least about 85-90%, preferably about 95% or more identical. Hence, all parts of a humanized antibody, except possibly the CDRs, are substantially identical to corresponding parts of natural human immunoglobulin sequences. Thus, a "humanized antibody" is an antibody comprising a humanized light chain and a humanized heavy chain immunoglobulin. A donor antibody is said to be "humanized", by the process of "humanization", because the resultant humanized antibody is expected to bind to the same antigen as the donor antibody that provides the CDRs. Reference herein to "humanized" includes reference to an antibody deimmunized to a particular host, in this case, a human host.

It will be understood that the deimmunized antibodies may have additional conservative amino acid substitutions which have substantially no effect on antigen binding or other immunoglobulin functions. Exemplary conservative substitutions may be made according to Table 1.

**TABLE 1**

| **ORIGINAL RESIDUE** | **EXEMPLARY SUBSTITUTIONS** |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

Exemplary methods which may be employed to produce deimmunized antibodies are described, for example, in Richmann *et al*., 1988, *supra*; European Patent No. 0 239 400; U.S. Patent No. 6,056,957, U.S. Patent No. 6,180,370, U.S. Patent No. 6,180,377.

Thus, the present description contemplates a deimmunized antibody molecule having specificity for an epitope recognized by a monoclonal antibody to a TLR such as TLR-2 or TLR-4 wherein at least one of the CDRs of the variable domain of said deimmunized antibody is derived from the said monoclonal antibody to said TLR and the remaining immunoglobulin-derived parts of the deimmunized antibody molecule are derived from an immunoglobulin or an analog thereof from the host for which the antibody is to be deimmunized.

The development of a deimmunized antibody involves manipulation of the framework region of a non-human antibody.

The antibodies disclosed herein extend to mutants and derivatives of the subject antibodies but which still retain specificity for TLR.

The terms "mutant" or "derivatives" includes one or more amino acid substitutions, additions and/or deletions.

As used herein, the term "CDR" includes CDR structural loops which covers to the three light chain and the three heavy chain regions in the variable portion of an antibody framework region which bridge β strands on the binding portion of the molecule. These loops have characteristic canonical structures (Chothia et al., J. Mol. Biol. 196: 901, 1987; Chothia et al., J. Mol. Biol. 227: 799, 1992).

By "framework region" is meant region of an immunoglobulin light or heavy chain variable region, which is interrupted by three hypervariable regions, also called CDRs. The extent of the framework region and CDRs have been precisely defined (see, for example, Kabat et al., "Sequences of Proteins of Immunological Interest", U.S. Department of Health and Human Sciences, 1983). The sequences of the framework regions of different light or heavy chains are relatively conserved within a species. As used herein, a "human framework region" is a framework region that is substantially identical (about 85% or more, usually 90-95% or more) to the framework region of a naturally occurring human immunoglobulin. The framework region of an antibody, that is the combined framework regions of the constituent light and heavy chains, serves to position and align the CDRs. The CDRs are primarily responsible for binding to an epitope of the TLR.

As used herein, the term "heavy chain variable region" means a polypeptide which is from about 110 to 125 amino acid residues in length, the amino acid sequence of which corresponds to that of a heavy chain of a monoclonal antibody of the invention, starting from the amino-terminal (N-terminal) amino acid residue of the heavy chain. Likewise, the term "light chain variable region" means a polypeptide which is from about 95 to 130 amino acid residues in length, the amino acid sequence of which corresponds to that of a light chain of a monoclonal antibody of the invention, starting from the N-terminal amino acid residue of the light chain. Full-length immunoglobulin "light chains" (about 25 Kd or 214 amino acids) are encoded by a variable region gene at the NH₂-terminus (about 110 amino acids) and a κ or λ constant region gene at the COOH-terminus. Full-length immunoglobulin "heavy chains" (about 50 Kd or 446 amino acids), are similarly encoded by a variable region gene (about 116 amino acids) and one of the other aforementioned constant region genes, e.g. γ (encoding about 330 amino acids).

The term "immunoglobulin" or "antibody" is used herein to refer to a protein consisting of one or more polypeptides substantially encoded by immunoglobulin genes. The recognized immunoglobulin genes include the κ, λ, α. γ (IgG₁, IgG₂, IgG₃, IgG₄), δ. ε and µ constant region genes, as well as the myriad immunoglobulin variable region genes. One form of immunoglobulin constitutes the basic structural unit of an antibody. This form is a tetramer and consists of two identical pairs of immunoglobulin chains, each pair having one light and one heavy chain. In each pair, the light and heavy chain variable regions are together responsible for binding to an antigen, and the constant regions are responsible for the antibody effector functions. In addition to antibodies, immunoglobulins may exist in a variety of other forms including, for example, Fv, Fab, Fab' and (Fab')₂.

The present invention contemplates the use of fragments of monoclonal antibodies produced as disclosed herein including, for example, Fv, Fab, Fab' and F(ab')₂ fragments. Such fragments may be prepared by standard methods as for example described by Coligan *et al*. (1991-1997, *supra*).

The present methods disclosed herein may use synthetic or recombinant antigen-binding molecules with the same or similar specificity as the monoclonal antibodies of the invention. Antigen-binding molecules of this type may comprise a synthetic stabilized Fv fragment. Exemplary fragments of this type include single chain Fv fragments (sFv, frequently termed scFv) in which a peptide linker is used to bridge the N terminus or C terminus of a V*_{H}* domain with the C terminus or N-terminus, respectively, of a V*_{L}* domain. ScFv lack all constant parts of whole antibodies and are not able to activate complement. Suitable peptide linkers for joining the V*_{H}* and V*_{L}* domains are those which allow the V*_{H}* and V*_{L}* domains to fold into a single polypeptide chain having an antigen binding site with a three dimensional structure similar to that of the antigen binding site of a whole antibody from which the Fv fragment is derived. Linkers having the desired properties may be obtained by the method disclosed in U.S. Patent No 4,946,778. However, in some cases a linker is absent. ScFvs may be prepared, for example, in accordance with methods outlined in Krebber et al. (J. Immunol. Methods 201(1): 35-55,1997). Alternatively, they may be prepared by methods described in U.S. Patent No 5,091,513, European Patent No 239,400 or the articles by Winter and Milstein (Nature 349: 293, 1991) and Plückthun et al. (In Antibody engineering: A practical approach, 203-252, 1996).

Alternatively, the synthetic stabilized Fv fragment comprises a disulphide stabilized Fv (dsFv) in which cysteine residues are introduced into the V*_{H}* and V*_{L}* domains such that in the fully folded Fv molecule the two residues will form a disulphide bond therebetween. Suitable methods of producing dsFv are described, for example, in (Glockshuber et al., Biochem. 29: 1363-1367, 1990; Reiter et al., J. Biol. Chem. 269: 18327-18331, 1994; Reiter et al., Biochem. 33: 5451-5459, 1994; Reiter et al., Cancer Res. 54: 2714-2718, 1994; Webber et al., Mol. Immunol. 32: 249-258, 1995).

Also contemplated as synthetic or recombinant antigen-binding molecules are single variable region domains (termed dAbs) as, for example, disclosed in (Ward et al., Nature 341: 544-546, 1989; Hamers-Casterman et al., Nature 363: 446-448; 1993; Davies & Riechmann, FEBS Lett. 339: 285-290, 1994).

Alternatively, the synthetic or recombinant antigen-binding molecule may comprise a "minibody". In this regard, minibodies are small versions of whole antibodies, which encode in a single chain the essential elements of a whole antibody. Suitably, the minibody is comprised of the V*_{H}* and V*_{L}* domains of a native antibody fused to the hinge region and CH3 domain of the immunoglobulin molecule as, for example, disclosed in U.S. Patent No 5,837,821.

As a further alternative, the synthetic or recombinant antigen binding molecule may comprise non-immunoglobulin derived, protein frameworks. For example, reference may be made to (Ku & Schutz, Proc. Natl. Acad. Sci. USA 92: 6552-6556, 1995) which discloses a four-helix bundle protein cytochrome b562 having two loops randomized to create CDRs, which have been selected for antigen binding.

The synthetic or recombinant antigen-binding molecule may be multivalent (i.e. having more than one antigen binding site). Such multivalent molecules may be specific for one or more antigens. Multivalent molecules of this type may be prepared by dimerization of two antibody fragments through a cysteinyl-containing peptide as, for example disclosed by (Adams et al., Cancer Res. 53: 4026-4034, 1993; Cumber et al., J. Immunol. 149: 120-126, 1992). Alternatively, dimerization may be facilitated by fusion of the antibody fragments to amphiphilic helices that naturally dimerize (Plüockthun, Biochem 31: 1579-1584, 1992) or by use of domains (such as leucine zippers jun and fos) that preferentially heterodimerize (Kostelny et al., J. Immunol. 148: 1547-1553, 1992). Multivalent antibodies are useful, for example, in detecting different forms of TLRs such as TLR-2 and TLR-4.

Yet another useful source of compounds useful in modulating TLR activity is a chemically modified ligand such as a cytokine or other activator of TLR which may then in turn activate or inhibit a TLR pathway.

In addition, compounds can be selected which interrupt or antagonize or agonize the interaction between a TLR and its ligand.

Analogs of proteinaceous molecules (e.g. ligands of a TLR) contemplated herein include but are not limited to modification to side chains, incorporating of unnatural amino acids and/or their derivatives during peptide, polypeptide or protein synthesis and the use of crosslinkers and other methods which impose conformational constraints on the proteinaceous molecule or their analogs.

Examples of side chain modifications contemplated herein include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH₄.

The guanidine group of arginine residues may be modified by the formation of heterocyclic condensation products with reagents such as 2,3-butanedione, phenylglyoxal and glyoxal.

The carboxyl group may be modified by carbodiimide activation *via* O-acylisourea formation followed by subsequent derivitization, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. A list of unnatural amino acid, contemplated herein is shown in Table 2.

**TABLE 2**

| ***Codes for non-conventional amino acids*** | | | |
|---|---|---|---|
| Non-conventional amino acid | Code | Non-conventional amino acid | Code |
| α-aminobutyric acid | Abu | L-N-methylalanine | Nmala |
| α-amino-α-methylbutyrate | Mgabu | L-N-methylarginine | Nmarg |
| aminocyclopropane-carboxylate | Cpro | L-N-methylasparagine | Nmasn |
| | | L-N-methylaspartic acid | Nmasp |
| aminoisobutyric acid | Aib | L-N-methylcysteine | Nmcys |
| aminonorbomyl-carboxylate | Norb | L-N-methylglutamine | Nmgln |
| | | L-N-methylglutamic acid | Nmglu |
| cyclohexylalanine | Chexa | L-Nmethylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmom |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dom | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α-methyl-aminoisobutyrate | Maib |
| D-valine | Dval | α-methyl-γ-aminobutyrate | Mgabu |
| D-α-methylalanine | Dmala | α-methylcyclohexylalanine | Mchexa |
| D-α-methylarginine | Dmarg | α-methylcylcopentylalanine | Mcpen |
| D-α-methylasparagine | Dmasn | α-methyl-α-napthylalanine | Manap |
| D-α-methylaspartate | Dmasp | α-methylpenicillamine | Mpen |
| D-α-methylcysteine | Dmcys | N-(4-aminobutyl)glycine | Nglu |
| D-α-methylglutamine | Dmgln | N-(2-aminoethyl)glycine | Naeg |
| D-α-methylhistidine | Dmhis | N-(3-aminopropyl)glycine | Nom |
| D-α-methylisoleucine | Dmile | N-amino-α-methylbutyrate | Nmaabu |
| D-α-methylleucine | Dmleu | α-napthylalanine | Anap |
| D-α-methyllysine | Dmlys | N-benzylglycine | Nphe |
| D-α-methylmethionine | Dmmet | N-(2-carbamylethyl)glycine | Ngln |
| D-α-methylornithine | Dmom | N-(carbamylmethyl)glycine | Nasn |
| D-α-methylphenylalanine | Dmphe | N-(2-carboxyethyl)glycine | Nglu |
| D-α-methylproline | Dmpro | N-(carboxymethyl)glycine | Nasp |
| D-α-methylserine | Dmser | N-cyclobutylglycine | Ncbut |
| D-α-methylthreonine | Dmthr | N-cycloheptylglycine | Nchep |
| D-α-methyltryptophan | Dmtrp | N-cyclohexylglycine | Nchex |
| D-α-methyltyrosine | Dmty | N-cyclodecylglycine | Ncdec |
| D-α-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-γ-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p*-hydroxyphenyl)glycine | Nhtyr |
| L-*t*-butylglycine | Thug | N-(thiomethyl)glycine | Ncys |
| L-ethylglycine | Etg | penicillamine | Pen |
| L-homophenylalanine | Hphe | L-α-methylalanine | Mala |
| L-α-methylarginine | Marg | L-α-methylasparagine | Masn |
| L-α-methylaspartate | Masp | L-α-methyl-*t*-butylglycine | Mtbug |
| L-α-methylcysteine | Mcys | L-methylethylglycine | Metg |
| L-α-methylglutamine | Mgln | L-α-methylglutamate | Mglu |
| L-α-methylhistidine | Mhis | L-α-methylhomophenylalanine | Mhphe |
| L-α-methylisoleucine | Mile | N-(2-methylthioethyl)glycine | Nmet |
| L-α-methylleucine | Mleu | L-α-methyllysine | Mlys |
| L-α-methylmethionine | Mmet | L-α-methylnorleucine | Mnle |
| L-α-methylnorvaline | Mnva | L-α-methylornithine | Mom |
| L-α-methylphenylalanine | Mphe | L-α-methylproline | Mpro |
| L-α-methylserine | Mser | L-α-methylthreonine | Mthr |
| L-α-methyltryptophan | Mtrp | L-α-methyltyrosine | Mtyr |
| L-α-methylvaline | Mval | L-N-methylhomophenylalanine | Nmhphe |
| N-(N-(2,2-diphenylethyl) | Nnbhm | N-(N-(3,3-diphenylpropyl) | Nnbhe |
| carbamylmethyl)glycine | | carbamylmethyl)glycine | |
| 1-carboxy-1-(2,2-diphenylethylamino)cyclopropane | Nmbc | | |

Crosslinkers can be used, for example, to stabilize 3D conformations, using homo- bifunctional crosslinkers such as the bifunctional imido esters having (CH₂)ₙ spacer groups with n=1 to n=6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of C_{α} and N _{α}-methylamino acids, introduction of double bonds between C_{α} and C_{β} atoms of amino acids and the formation of cyclic peptides or analogs by introducing covalent bonds such as forming an amide bond between the N and C termini, between two side chains or between a side chain and the N or C terminus.

Accordingly, the present description contemplates any compound which binds or otherwise interacts with a TLR, such as TLR-2 or TLR-4, or a component of a TLR signaling pathway resulting in potentiation, activation or up-regulation or antagonism or down-regulation of the TLR or TLR signaling pathway.

Another useful group of compounds disclosed herein is a mimetic. The terms "peptide mimetic", "target mimetic" or "mimetic" are intended to refer to a substance which has some chemical similarity to the target but which antagonizes or agonizes or mimics the target. The target in this case may be a TLR ligand. A peptide mimetic may be a peptide-containing molecule that mimics elements of protein secondary structure (Johnson et al., "Peptide Turn Mimetics" in Biotechnology and Pharmacy, Pezzuto et al., Eds., Chapman and Hall, New York, 1993). The underlying rationale behind the use of peptide mimetics is that the peptide backbone of proteins exists chiefly to orient amino acid side chains in such a way as to facilitate molecular interactions such as those of antibody and antigen, enzyme and substrate or scaffolding proteins. A peptide mimetic is designed to permit molecular interactions similar to the natural molecule. Peptide or non-peptide mimetics may be useful, for example, to activate a TLR or TLR pathway or to competitively inhibit a TLR. Preferred TLRs in this instance are TLR-2 and TLR-4.

Again, the compounds disclosed herein may be selected to interact with a target alone or single or multiple compounds may be used to affect multiple targets. For example, multiple targets may include a TLR and the microorganism or virus itself.

The target TLR or fragment employed in screening assays may either be free in solution, affixed to a solid support, or borne on a cell surface. One method of drug screening utilizes eukaryotic or prokaryotic host cells which are stably transformed with recombinant polynucleotides expressing the TLR or fragment, preferably in competitive binding assays. Such cells, either in viable or fixed form, can be used for standard binding assays. One may measure, for example, the formation of complexes between a TLR or fragment and the agent being tested, or examine the degree to which the formation of a complex between a TLR or fragment and a ligand is aided or interfered with by the agent being tested.

A substance identified as a modulator of target function or gene activity may be a peptide or non-peptide in nature. Non-peptide "small molecules" are often preferred for many *in vivo* pharmaceutical uses. Accordingly, a mimetic or mimic of the substance (particularly if a peptide) may be designed for pharmaceutical use.

The designing of mimetics to a pharmaceutically active compound is a known approach to the development of pharmaceuticals based on a "lead" compound. This might be desirable where the active compound is difficult or expensive to synthesize or where it is unsuitable for a particular method of administration, e.g. peptides are unsuitable active agents for oral compositions as they tend to he quickly degraded by proteases in the alimentary canal. Mimetic design, synthesis and testing is generally used to avoid randomly screening large numbers of molecules for a target property.

There are several steps commonly taken in the design of a mimetic from a compound having a given target property. First, the particular parts of the compound that are critical and/or important in determining the target property are determined. In the case of a peptide, this can be done by systematically varying the amino acid residues in the peptide, e.g. by substituting each residue in turn. Alanine scans of peptides are commonly used to refine such peptide motifs. These parts or residues constituting the active region of the compound are known as its "pharmacophore".

Once the pharmacophore has been found, its structure is modeled according to its physical properties, e.g. stereochemistry, bonding, size and/or charge, using data from a range of sources, e.g. spectroscopic techniques, x-ray diffraction data and NMR. Computational analysis, similarity mapping (which models the charge and/or volume of a pharmacophore, rather than the bonding between atoms) and other techniques can be used in this modeling process.

In a variant of this approach, the three-dimensional structure of a TLR and its ligand are modeled. This can be especially useful where the TLR and/or its ligand change conformation on binding, allowing the model to take account of this in the design of the mimetic. Modeling can be used to generate inhibitors which interact with the linear sequence or a three-dimensional configuration.

A template molecule is then selected onto which chemical groups which mimic the pharmacophore can be grafted. The template molecule and the chemical groups grafted onto it can conveniently be selected so that the mimetic is easy to synthesize, is likely to be pharmacologically acceptable, and does not degrade *in vivo,* while retaining the biological activity of the lead compound. Alternatively, where the mimetic is peptide-based, further stability can be achieved by cyclizing the peptide, increasing its rigidity. The mimetic or mimetics found by this approach can then be screened to see whether they have the target property, or to what extent they exhibit it. Further optimization or modification can then be carried out to arrive at one or more final mimetics for *in vivo* or clinical testing.

The goal of rational drug design is to produce structural analogs of biologically active polypeptides of interest or of small molecules with which they interact (e.g. agonists, antagonists, inhibitors or enhancers) in order to fashion drugs which are, for example, more active or stable forms of the polypeptide, or which, e.g. enhance or interfere with the function of a polypeptide *in vivo.* See, e.g. Hodgson (Bio/Technology 9: 19-21, 1991). In one approach, one first determines the three-dimensional structure of a TLR ligand by x-ray crystallography, by computer modeling or most typically, by a combination of approaches. Useful information regarding the structure of a TLR ligand may also be gained by modeling based on the structure of homologous proteins. An example of rational drug design is the development of HIV protease inhibitors (Erickson et al., Science 249: 527-533, 1990). In addition, target molecules may be analyzed by an alanine scan (Wells, Methods Enzymol. 202: 2699-2705, 1991). In this technique, an amino acid residue is replaced by Ala and its effect on the peptide's activity is determined. Each of the amino acid residues of the peptide is analyzed in this manner to determine the important regions of the peptide.

Proteomics may be also be used to screen for the differential production of components of the TLR signaling pathway or of particular TLRs such as TLR-2 and TLR-4 in response to different physiological conditions and/or in the presence of candidate drugs.

It is also possible to isolate a TLR-specific or TLR ligand-specific antibody (such as by the method described above) and then to solve its crystal structure. In principle, this approach yields a pharmacophore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced banks of peptides. Selected peptides would then act as the pharmacophore.

The present description and methods and use of the invention extends to a genetic approach to up-regulating or down-regulating expression of a gene encoding a TLR, such as TLR-2 or TLR-4, or up- or down-regulating a compound in the TLR signaling pathway. Generally, it is more convenient to use genetic means to induce gene silencing such as pre- or post-transcriptional gene silencing. However, the general techniques can be used to up-regulate expression such as by increasing gene copy numbers or antagonizing inhibitors of gene expression.

The terms "nucleic acids", "nucleotide" and "polynucleotide" include RNA, cDNA, genomic DNA, synthetic forms and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog (such as the morpholine ring), internucleotide modifications such as uncharged linkages (e.g. methyl phosphonates, phosphotriesters, phosphoamidates, carbamates, etc.), charged linkages (e.g. phosphorothioates, phosphorodithioates, etc.), pendent moieties (e.g. polypeptides), intercalators (e.g. acridine, psoralen, etc.), chelators, alkylators and modified linkages (e.g. α-anomeric nucleic acids, etc.). Also included are synthetic molecules that mimic polynucleotides in their ability to bind to a designated sequence *via* hydrogen binding and other chemical interactions. Such molecules are known in the art and include, for example, those in which peptide linkages substitute for phosphate linkages in the backbone of the molecule.

Antisense polynucleotide sequences, for example, are useful in silencing transcripts of TLR genes, such as TLR-2 or TLR-4 gene transcripts. Expression of such an antisense construct within a cell interferes with TLR gene transcription and/or translation. Furthermore, co-suppression and mechanisms to induce RNAi or siRNA may also be employed. Alternatively, antisense or sense molecules may be directly administered. In this latter embodiment, the antisense or sense molecules may be formulated in a composition and then administered by any number of means to target cells.

A variation on antisense and sense molecules involves the use of morpholinos, which are oligonucleotides composed of morpholine nucleotide derivatives and phosphorodiamidate linkages (for example, Summerton and Weller, Antisense and Nucleic Acid Drug Development 7: 187-195, 1997). Such compounds are injected into embryos and the effect of interference with mRNA is observed.

The present description discloses compounds such as oligonucleotides and similar species for use in modulating the function or effect of nucleic acid molecules such as those encoding a TLR, such as TLR-2 or TLR-4, i.e. the oligonucleotides induce pre-transcriptional or post-transcriptional gene silencing. This is accomplished by providing oligonucleotides which specifically hybridize with one or more nucleic acid molecules encoding the TLR gene transcription. The oligonucleotides may be provided directly to a cell or generated within the cell. As used herein, the terms "target nucleic acid" and "nucleic acid molecule encoding a TLR gene transcript" have been used for convenience to encompass DNA encoding the TLR, RNA (including pre-mRNA and mRNA or portions thereof) transcribed from such DNA, and also cDNA derived from such RNA. The hybridization of a compound of the subject invention with its target nucleic acid is generally referred to as "antisense". Consequently, the preferred mechanism believed to be included in the practice of some preferred embodiments of the invention is referred to herein as "antisense inhibition." Such antisense inhibition is typically based upon hydrogen bonding-based hybridization of oligonucleotide strands or segments such that at least one strand or segment is cleaved, degraded, or otherwise rendered inoperable. In this regard, it is presently preferred to target specific nucleic acid molecules and their functions for such antisense inhibition.

The functions of DNA to be interfered with can include replication and transcription. Replication and transcription, for example, can be from an endogenous cellular template, a vector, a plasmid construct or otherwise. The functions of RNA to be interfered with can include functions such as translocation of the RNA to a site of protein translation, translocation of the RNA to sites within the cell which are distant from the site of RNA synthesis, translation of protein from the RNA, splicing of the RNA to yield one or more RNA species, and catalytic activity or complex formation involving the RNA which may be engaged in or facilitated by the RNA. In one example, the result of such interference with TLR transcript function is reduced levels of the TLR. As referred to herein "modulation" and "modulation of expression" mean either an increase (stimulation) or a decrease (inhibition) in the amount or levels of a nucleic acid molecule encoding the gene, e.g., DNA or RNA. Inhibition is often the preferred form of modulation of expression and mRNA is often a preferred target nucleic acid.

As used herein, "hybridization" means the pairing of complementary strands of oligomeric compounds. In the present invention, the preferred mechanism of pairing involves hydrogen bonding, which may be Watson-Crick, Hoogsteen or reversed Hoogsteen hydrogen bonding, between complementary nucleoside or nucleotide bases (nucleobases) of the strands of oligomeric compounds. For example, adenine and thymine are complementary nucleobases which pair through the formation of hydrogen bonds. Hybridization can occur under varying circumstances.

An antisense compound is specifically hybridizable when binding of the compound to the target nucleic acid interferes with the normal function of the target nucleic acid to cause a loss of activity, and there is a sufficient degree of complementarity to avoid non-specific binding of the antisense compound to non-target nucleic acid sequences under conditions in which specific binding is desired, i.e. under physiological conditions in the case of *in vivo* assays or therapeutic treatment, and under conditions in which assays are performed in the case of *in vitro* assays.

"Complementary" as used herein, refers to the capacity for precise pairing between two nucleobases of an oligomeric compound. For example, if a nucleobase at a certain position of an oligonucleotide (an oligomeric compound), is capable of hydrogen bonding with a nucleobase at a certain position of a target nucleic acid, said target nucleic acid being a DNA, RNA, or oligonucleotide molecule, then the position of hydrogen bonding between the oligonucleotide and the target nucleic acid is considered to be a complementary position. The oligonucleotide and the further DNA, RNA, or oligonucleotide molecule are complementary to each other when a sufficient number of complementary positions in each molecule are occupied by nucleobases which can hydrogen bond with each other. Thus, "specifically hybridizable" and "complementary" are terms which are used to indicate a sufficient degree of precise pairing or complementarity over a sufficient number of nucleobases such that stable and specific binding occurs between the oligonucleotide and a target nucleic acid.

According to the present description, compounds include antisense oligomeric compounds, antisense oligonucleotides, ribozymes, external guide sequence (EGS) oligonucleotides, alternate splicers, primers, probes, and other oligomeric compounds which hybridize to at least a portion of the target nucleic acid. As such, these compounds may be introduced in the form of single-stranded, double-stranded, circular or hairpin oligomeric compounds and may contain structural elements such as internal or terminal bulges or loops. Once introduced to a system, the compounds may elicit the action of one or more enzymes or structural proteins to effect modification of the target nucleic acid. One non-limiting example of such an enzyme is RNAse H, a cellular endonuclease which cleaves the RNA strand of an RNA:DNA duplex. It is known in the art that single-stranded antisense compounds which are "DNA-like" elicit RNAse H. Activation of RNase H, therefore, results in cleavage of the RNA target, thereby greatly enhancing the efficiency of oligonucleotide-mediated inhibition of gene expression. Similar roles have been postulated for other ribonucleases such as those in the RNase III and ribonuclease L family of enzymes.

While the preferred form of antisense compound is a single-stranded antisense oligonucleotide, in many species the introduction of double-stranded structures, such as double-stranded RNA (dsRNA) molecules, has been shown to induce potent and specific antisense-mediated reduction of the function of a gene or its associated gene products. This phenomenon occurs in both plants and animals.

As used herein, the term "oligomeric compound" refers to a polymer or oligomer comprising a plurality of monomeric units. As used herein, the term "oligonucleotide" refers to an oligomer or polymer of ribonucleic acid (RNA) or deoxyribonucleic acid (DNA) or mimetics, chimeras, analogs and homologs thereof. This term includes oligonucleotides composed of naturally occurring nucleobases, sugars and covalent internucleoside (backbone) linkages as well as oligonucleotides having non-naturally occurring portions which function similarly. Such modified or substituted oligonucleotides are often preferred over native forms because of desirable properties such as, for example, enhanced cellular uptake, enhanced affinity for a target nucleic acid and increased stability in the presence of nucleases.

While oligonucleotides are a preferred form of the compounds (agents) for use according to this invention, the present invention comprehends other families of compounds as well, including but not limited to oligonucleotide analogs and mimetics such as those herein described.

The open reading frame (ORF) or "coding region" which is known in the art to refer to the region between the translation initiation codon and the translation termination codon, is a region which may be effectively targeted. Within the context of the present invention, one region is the intragenic region encompassing the translation initiation or termination codon of the open reading frame (ORF) of a gene.

Other target regions include the 5' untranslated region (5'UTR), known in the art to refer to the portion of an mRNA in the 5' direction from the translation initiation codon, and thus including nucleotides between the 5' cap site and the translation initiation codon of an mRNA (or corresponding nucleotides on the gene), and the 3' untranslated region (3'UTR), known in the art to refer to the portion of an mRNA in the 3' direction from the translation termination codon, and thus including nucleotides between the translation termination codon and 3' end of an mRNA (or corresponding nucleotides on the gene). The 5' cap site of an mRNA comprises an N7-methylated guanosine residue joined to the 5'-most residue of the mRNA *via* a 5'-5' triphosphate linkage. The 5' cap region of an mRNA is considered to include the 5' cap structure itself as well as the first 50 nucleotides adjacent to the cap site. It is also preferred to target the 5' cap region.

Although some eukaryotic mRNA transcripts are directly translated, many contain one or more regions, known as "introns", which are excised from a transcript before it is translated. The remaining (and, therefore, translated) regions are known as "exons" and are spliced together to form a continuous mRNA sequence. Targeting splice sites, i.e. intron-exon junctions or exon-intron junctions, may also be particularly useful in situations where aberrant splicing is implicated in disease, or where an overproduction of a particular splice product is implicated in disease. Aberrant fusion junctions due to rearrangements or deletions are also preferred target sites. mRNA transcripts produced *via* the process of splicing of two (or more) mRNAs from different gene sources are known as "fusion transcripts". It is also known that introns can be effectively targeted using antisense compounds targeted to, for example, DNA or pre-mRNA.

As is known in the art, a nucleoside is a base-sugar combination. The base portion of the nucleoside is normally a heterocyclic base. The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn, the respective ends of this linear polymeric compound can be further joined to form a circular compound, however, linear compounds are generally preferred. In addition, linear compounds may have internal nucleobase complementarity and may, therefore, fold in a manner as to produce a fully or partially double-stranded compound. Within oligonucleotides, the phosphate groups are commonly referred to as forming the internucleoside backbone of the oligonucleotide. The normal linkage or backbone of RNA and DNA is a 3' to 5' phosphodiester linkage.

Specific examples of preferred antisense compounds useful in this invention include oligonucleotides containing modified backbones or non-natural internucleoside linkages. As defined in this specification, oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides.

Preferred modified oligonucleotide backbones containing a phosphorus atom therein include, for example, phosphorothioates, chiral phosphorothioates, phosphorodithioates, phosphotriesters, aminoalkylphosphotriesters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and boranophosphates having normal 3'-5' linkages, 2'-5' linked analogs of these, and those having inverted polarity wherein one or more internucleotide linkages is a 3' to 3', 5' to 5' or 2' to 2' linkage. Preferred oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included.

The antisense oligonucleotides may be administered by any convenient means including by inhalation, local or systemic means.

Alternatively, genetic constructs including DNA vaccines may be used to generate antisense molecules *in vivo.* Furthermore, many of the preferred features described above are appropriate for sense nucleic acid molecules or for gene therapy applications to promote levels of TLRs.

Following identification of an agent which potentiates or antagonizes a TLR or TLR pathway, it may be manufactured and/or used in a preparation, i.e. in the manufacture or formulation or a composition such as a medicament, pharmaceutical composition or drug. These may be administered to individuals in a method of treatment or prophylaxis of inection. Alternatively, they may be incorporated into a patch or slow release capsule or implant.

Also disclosed herein is a pharmaceutical composition, medicament, drug or other composition including a patch or slow release formulation comprising an agonist or antagonist of TLR activity or TLR gene expression or the activity or gene expression of a component of the TLR signaling pathway.

The present description contemplates a method comprising administration of such a composition to a subject such as for treatment or prophylaxis of an infection. Furthermore, the present description contemplates a method of making a pharmaceutical composition comprising admixing a compound of the instant invention with a pharmaceutically acceptable excipient, vehicle or carrier, and optionally other ingredients. Where multiple compositions are provided, then such compositions may be given simultaneously or sequentially. Sequential administration includes administration within nanoseconds, seconds, minutes, hours or days. Preferably, sequential administration is within seconds or minutes.

Multi-part including two-part pharmaceutical compositions or packs are also disclosed comprising multiple components such as those which potentiate or inhibit a TLR such as TLR-2 or TLR-4 together with anti-microbial or anti-viral agents. Such multi-part pharmaceutical compositions or packs maintain different agents or groups of agents separately. These are either dispensed separately or admixed prior to being dispensed.

The present description discloses a method for the treatment or prophylaxis of an infection in a subject, said method comprising administering to said subject an effective amount of a compound as described herein or a composition comprising same.

Thus in a further aspect, the present invention provides an agent which up-regulates or down-regulates the level of TLR-2 and/or TLR-4 for use in treating a subject infected with HCV or HBV wherein the agent down-regulates TLR-2 and TLR-4 in anti-HCV treatment and up-regulates TLR-2 in anti-HBV treatment, wherein said agent is selected from an antibody specific for TLR-2 or TLR-4 or an antigen-binding fragment thereof, a sense or antisense nucleic acid molecule to TLR-2 or TLR-4 and RNAi, siRNA, a ribozyme or a DNAzyme to TLR-2 or TLR-4.

Preferably, the subject is a mammal such as a human or laboratory test animal such as a mouse, rat, rabbit, guinea pig, hamster, zebrafish or amphibian or avian species such as a duck.

This disclosed method also includes providing a wild-type or mutant target gene function to a cell. This is particularly useful when generating an animal model. Alternatively, it may be part of a gene therapy approach. A target gene or a part of the gene may be introduced into the cell in a vector such that the gene remains extrachromosomal. In such a situation, the gene will be expressed by the cell from the extrachromosomal location. If a gene portion is introduced and expressed in a cell carrying a mutant target allele, the gene portion should encode a part of the target protein. Vectors for introduction of genes both for recombination and for extrachromosomal maintenance are known in the art and any suitable vector may be used. Methods for introducing DNA into cells such as electroporation calcium phosphate co-precipitation and viral transduction are known in the art.

Gene transfer systems known in the art may be useful in the practice of genetic manipulation. These include viral and non-viral transfer methods. A number of viruses have been used as gene transfer vectors or as the basis for preparing gene transfer vectors, including papovaviruses (e.g. SV40, Madzak et al., J. Gen. Virol. 73: 1533-1536, 1992), adenovirus (Berkner, Curr. Top. Microbiol. Immunol. 158: 39-66, 1992; Berkner et al., BioTechniques 6; 616-629, 1988; Gorziglia and Kapikian, J. Virol. 66: 4407-4412, 1992; Quantin et al., Proc. Natl. Acad. Sci. USA 89: 2581-2584, 1992; Rosenfeld et al., Cell 68: 143-155, 1992; Wilkinson et al., Nucleic Acids Res. 20: 2233-2239, 1992; Stratford-Perricaudet et al., Hum. Gene Ther. 1: 241-256, 1990; Schneider et al., Nature Genetics 18: 180-183, 1998), vaccinia virus (Moss, Curr. Top. Microbiol. Immunol. 158: 25-38, 1992; Moss, Proc. Natl. Acad. Sci. USA 93: 11341-11348, 1996), adeno-associated virus (Muzyczka, Curr. Top. Microbiol. Immunol. 158: 97-129, 1992; Ohi et al., Gene 89: 279-282, 1990; Russell and Hirata, Nature Genetics 18: 323-328, 1998), herpesviruses including HSV and EBV (Margolskee, Curr. Top., Microbiol. Immunol. 158: 67-95, 1992; Johnson et al., J. Virol. 66: 2952-2965, 1992; Fink et al., Hum. Gene Ther. 3: 11-19, 1992; Breakefield and Geller, Mol. Neurobiol. 1: 339-371, .1987; Freese et al., Biochem. Pharmacol. 40: 2189-2199, 1990; Fink et al., Ann. Rev. Neurosci. 19: 265-287, 1996), lentiviruses (Naldini et al., Science 272: 263-267, 1996), Sindbis and Semliki Forest virus (Berglund et al., Biotechnology 11: 916-920, 1993) and retroviruses of avian (Bandyopadhyay and Temin, Mol. Cell. Biol. 4: 749-754, 1984; Petropoulos et al., J. Viol. 66: 3391-3397, 1992], murine [Miller, Curr. Top. Microbiol. Immunol. 158: 1-24, 1992; Miller et al., Mol. Cell. Biol. 5: 431-437, 1985; Sorge et al., Mol. Cell. Biol. 4: 1730-1737, 1984; and Baltimore, J. Virol. 54: 401-407, 1985; Miller et al., J. Virol. 62: 4337-4345, 1988] and human [Shimada et al., J. Clin. Invest. 88: 1043-1047, 1991; Helseth et al., J. Virol. 64: 2416-2420, 1990; Page et al., J. Virol. 64: 5270-5276, 1990; Buchschacher and Panganiban, J. Virol. 66: 2731-2739, 1982] origin.

Non-viral gene transfer methods are known in the art such as chemical techniques including calcium phosphate co-precipitation, mechanical techniques, for example, microinjection, membrane fusion-mediated transfer *via* liposomes and direct DNA uptake and receptor-mediated DNA transfer. Viral-mediated gene transfer can be combined with direct *in vivo* gene transfer using liposome delivery, allowing one to direct the viralvectors to particular cells. Alternatively, the retroviral vector producer cell line can be injected into particular tissue. Injection of producer cells would then provide a continuous source of vector particles.

In an approach which combines biological and physical gene transfer methods, plasmid DNA of any size is combined with a polylysine-conjugated antibody specific to the adenovirus hexon protein and the resulting complex is bound to an adenovirus vector. The trimolecular complex is then used to infect cells. The adenovirus vector permits efficient binding, internalization and degradation of the endosome before the coupled DNA is damaged. For other techniques for the delivery of adenovirus based vectors, see U.S. Patent No. 5,691,198.

Liposome/DNA complexes have been shown to be capable of mediating direct *in vivo* gene transfer. While in standard liposome preparations the gene transfer process is non-specific, localized *in vivo* uptake and expression have been reported in tumor deposits, for example, following direct *in situ* administration.

If the polynucleotide encodes a sense or antisense polynucleotide or a ribozyme or DNAzyme, expression will produce the sense or antisense polynucleotide or ribozyme or DNAzyme. Thus, in this context, expression does not require that a protein product be synthesized. In addition to the polynucleotide cloned into the expression vector, the vector also contains a promoter functional in eukaryotic cells. The cloned polynucleotide sequence is under control of this promoter. Suitable eukaryotic promoters include those described above. The expression vector may also include sequences, such as selectable markers and other sequences described herein.

Cells which carry mutant target alleles (e.g. TLR-2 or TLR-4) or where one or both alleles are deleted or up-regulated can be used as model systems to study the effects of infection or other disease condition.

The compounds, agents, medicaments, nucleic acid molecules and other target antagonists or agonists disclosed herein can be formulated in pharmaceutical compositions which are prepared according to conventional pharmaceutical compounding techniques. See, for example, Remington's Pharmaceutical Sciences, 18th Ed. (1990, Mack Publishing, Company, Easton, PA, U.S.A.). The composition may contain the active agent or pharmaceutically acceptable salts of the active agent. These compositions may comprise, in addition to one of the active substances, a pharmaceutically acceptable excipient, carrier, buffer, stabilizer or other materials well known in the art. Such materials should be nontoxic and should not interfere with the efficacy of the active ingredient. The carrier may take a wide variety of forms depending on the form of preparation desired for administration, e.g. topical, intravenous, oral, intrathecal, epineural or parenteral.

For oral administration, the compounds can be formulated into solid or liquid preparations such as capsules, pills, tablets, lozenges, powders, suspensions or emulsions. In preparing the compositions in oral dosage form, any of the usual pharmaceutical media may be employed, such as, for example, water, glycols, oils, alcohols, flavoring agents, preservatives, coloring agents, suspending agents, and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are obviously employed. If desired, tablets may be sugar-coated or enteric-coated by standard techniques. The active agent can be encapsulated to make it stable to passage through the gastrointestinal tract while at the same time allowing for passage across the blood brain barrier. See for example, International Patent Publication No. WO 96/11698.

For parenteral administration, the compound may be dissolved in a pharmaceutical carrier and administered as either a solution of a suspension. Illustrative of suitable carriers are water, saline, dextrose solutions, fructose solutions, ethanol, or oils of animal, vegetative or synthetic origin. The carrier may also contain other ingredients, for example, preservatives, suspending agents, solubilizing agents, buffers and the like. When the compounds are being administered intrathecally, they may also be dissolved in cerebrospinal fluid.

The active agent is preferably administered in a therapeutically effective amount. The actual amount administered and the rate and time-course of administration will depend on the nature and severity of the condition being treated. Prescription of treatment, e.g. decisions on dosage, timing, etc. is within the responsibility of general practitioners or specialists and typically takes account of the disorder to be treated, the condition of the individual patient, the site of delivery, the method of administration and other factors known to practitioners. Examples of techniques and protocols can be found in Remington's Pharmaceutical Sciences, *supra*.

Alternatively, targeting therapies may be used to deliver the active agent more specifically to certain types of cell, by the use of targeting systems such as antibodies or cell specific ligands or specific nucleic acid molecules. Targeting may be desirable for a variety of reasons, e.g. if the agent is unacceptably toxic or if it would otherwise require too high a dosage or if it would not otherwise be able to enter the target cells.

Instead of administering these agents directly, they could be produced in the target cell, e.g. in a viral vector such as described above or in a cell based delivery system such as described in U.S. Patent No. 5,550,050 and International Patent Publication Nos. WO 92/19195, WO 94/25503, WO 95/01203, WO 95/05452, WO 96/02286, WO 96/02646, WO 96/40871, WO 96/40959 and WO 97/12635. The vector could be targeted to the target cells. The cell based delivery system is designed to be implanted in a patient's body at the desired target site and contains a coding sequence for the target agent. Alternatively, the agent could be administered in a precursor form for conversion to the active form by an activating agent produced in, or targeted to, the cells to be treated. See, for example, European Patent Application No. 0 425 731 A and International Patent Publication No. WO 90/07936.

The present invention further provides *in vitro* diagnostic methods such as to determine whether a subject will respond to an anti-HCV treatment or an anti-HBV treatment, for monitoring a response to treatment of a subject against HCV or HBV infection and for predicting the outcome of a therapeutic protocol directed against infection by HCV or HBV. These methods comprise determining the level of TLR-2 and/or TLR-4.

Immunological based TLR detection protocols may take a variety of forms. For example, a plurality of antibodies may be immobilized in an array each with different specificities to particular TLRs or monocytes or hepatocytes comprising TLRs. Cells from a biopsy are then brought into contact with the antibody array and a diagnosis may be made as to the level and type of TLRs elevated or down-regulated on the cell.

Other more conventional assays may also be conducted such as by ELISA, Western blot analysis, immunoprecipitation analysis, immunofluorescence analysis, immunochemistry analysis or FACS analysis.

The present description discloses a method of detecting a TLR or cell comprising same or fragment, variant or derivative thereof comprising contacting the sample with an antibody or fragment or derivative thereof and detecting the level of a complex comprising said antibody and the TLR or fragment, variant or derivative thereof compared to normal controls wherein altered levels of the TLR is indicative of the presence or absence of infection or other disease condition.

Preferably, the TLR is TLR-2 and/or TLR-4.

As discussed above, any suitable technique for determining formation of the complex may be used. For example, an antibody as disclosed herein, having a reporter molecule associated therewith, may be utilized in immunoassays. Such immunoassays include but are not limited to radioimmunoassays (RIAs), enzyme-linked immunosorbent assays (ELISAs), immunochromatographic techniques (ICTs) and Western blotting which are well known to those of skill in the art. For example, reference may be made to Coligan *et al*., 1991-1997, *supra* which discloses a variety of immunoassays which may be used in methods disclosed herein. Immunoassays may include competitive assays. It will be understood that the present invention encompasses qualitative and quantitative immunoassays.

Suitable immunoassay techniques are described, for example, in U.S. Patent Nos. 4,016,043, 4,424,279 and 4,018,653. These include both single-site and two-site assays of the non-competitive types, as well as the traditional competitive binding assays. These assays also include direct binding of a labeled antigen-binding molecule to a target antigen. The antigen in this case is the TLR or a fragment thereof.

Two-site assays are particularly favoured for use in the methods disclosed herein. A number of variations of these assays exist, all of which are intended to be encompassed by the present invention. Briefly, in a typical forward assay, an unlabeled antigen-binding molecule such as an unlabeled antibody is immobilized on a solid substrate and the sample to be tested brought into contact with the bound molecule. After a suitable period of incubation, for a period of time sufficient to allow formation of an antibody-antigen complex, another antigen-binding molecule, suitably a second antibody specific to the antigen, labeled with a reporter molecule capable of producing a detectable signal is then added and incubated, allowing time sufficient for the formation of another complex of antibody-antigen-labeled antibody. Any unreacted material is washed away and the presence of the antigen is determined by observation of a signal produced by the reporter molecule. The results may be either qualitative, by simple observation of the visible signal, or may be quantitated by comparing with a control sample containing known amounts of antigen. Variations on the forward assay include a simultaneous assay, in which both sample and labeled antibody are added simultaneously to the bound antibody. These techniques are well known to those skilled in the art, including minor variations as will be readily apparent.

In the typical forward assay, a first antibody having specificity for the antigen or antigenic parts thereof is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well known in the art and generally consist of cross-linking covalently binding or physically adsorbing, the polymer-antibody complex is washed in preparation for the test sample. An aliquot of the sample to be tested is then added to the solid phase complex and incubated for a period of time sufficient and under suitable conditions to allow binding of any antigen present to the antibody. Following the incubation period, the antigen-antibody complex is washed and dried and incubated with a second antibody specific for a portion of the antigen. The second antibody has generally a reporter molecule associated therewith that is used to indicate the binding of the second antibody to the antigen. The amount of labeled antibody that binds, as determined by the associated reporter molecule, is proportional to the amount of antigen bound to the immobilized first antibody.

An alternative method involves immobilizing the antigen in the biological sample and then exposing the immobilized antigen to specific antibody that may or may not be labeled with a reporter molecule. Depending on the amount of target and the strength of the reporter molecule signal, a bound antigen may be detectable by direct labelling with the antibody. Alternatively, a second labeled antibody, specific to the first antibody is exposed to the target-first antibody complex to form a target-first antibody-second antibody tertiary complex. The complex is detected by the signal emitted by the reporter molecule.

From the foregoing, it will be appreciated that the reporter molecule associated with the antigen-binding molecule may include the following:-
(a) direct attachment of the reporter molecule to the antibody;
(b) indirect attachment of the reporter molecule to the antibody; i.e., attachment of the reporter molecule to another assay reagent which subsequently binds to the antibody; and
(c) attachment to a subsequent reaction product of the antibody.

The reporter molecule may be selected from a group including a chromogen, a catalyst, an enzyme, a fluorochrome, a chemiluminescent molecule, a paramagnetic ion, a lanthanide ion such as Europium (Eu³⁴), a radioisotope including other nuclear tags and a direct visual label.

In the case of a direct visual label, use may be made of a colloidal metallic or non-metallic particle, a dye particle, an enzyme or a substrate, an organic polymer, a latex particle, a liposome, or other vesicle containing a signal producing substance and the like.

A large number of enzymes suitable for use as reporter molecules is disclosed in U.S. Patent Nos. U.S. 4,366,241, U.S. 4,843,000, and U.S. 4,849,338. Suitable useful enzymes include alkaline phosphatase, horseradish peroxidase, luciferase, β-galactosidase, glucose oxidase, lysozyme, malate dehydrogenase and the like. The enzymes may be used alone or in combination with a second enzyme that is in solution.

Suitable fluorochromes include, but are not limited to, fluorescein isothiocyanate (FITC), tetramethylrhodamine isothiocyanate (TRITC), R-Phycoerythrin (RPE), and Texas Red. Other exemplary fluorochromes include those discussed by International Patent Publication No. WO 93/06121. Reference also may be made to the fluorochromes described in U.S. Patent Nos. 5,573,909 and 5,326,692. Alternatively, reference may be made to the fluorochromes described in U.S. Patent Nos. 5,227,487, 5,274,113, 5,405,975, 5,433,896, 5,442,045, 5,451,663, 5,453,517, 5,459,276, 5,516,864, 5,648,270 and 5,723,218.

In the case of an enzyme immunoassay, an enzyme is conjugated to the second antibody, generally by means of glutaraldehyde or periodate. As will be readily recognized, however, a wide variety of different conjugation techniques exist which are readily available to the skilled artisan. The substrates to be used with the specific enzymes are generally chosen for the production of, upon hydrolysis by the corresponding enzyme, a detectable colour change. Examples of suitable enzymes include those described *supra*. It is also possible to employ fluorogenic substrates, which yield a fluorescent product rather than the chromogenic substrates noted above. In all cases, the enzyme-labeled antibody is added to the first antibody-antigen complex, allowed to bind, and then the excess reagent washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of antigen which was present in the sample.

Alternately, fluorescent compounds, such as fluorescein, rhodamine and the lanthanide, europium (EU), may be chemically coupled to antibodies without altering their binding capacity. When activated by illumination with light of a particular wavelength, the fluorochrome-labeled antibody adsorbs the light energy, inducing a state of excitability in the molecule, followed by emission of the light at a characteristic colour visually detectable with a light microscope. The fluorescent-labeled antibody is allowed to bind to the first antibody-antigen complex. After washing off the unbound reagent, the remaining tertiary complex is then exposed to light of an appropriate wavelength. The fluorescence observed indicates the presence of the antigen of interest. Immunofluorometric assays (IFMA) are well established in the art and are particularly useful for the present method. However, other reporter molecules, such as radioisotope, chemiluminescent or bioluminescent molecules may also be employed.

Monoclonal antibodies to a TLR may also be used in ELISA-mediated detection of the TLR. This may be undertaken in any number of ways such as immobilizing anti-TLR antibodies to a solid support and contacting these with PBMCs and/or liver cells. Labeled anti-TLR antibodies are then used to detect immobilized TLR. Alternatively, antibodies to other PBMC or liver cell surface markers are used. This assay may be varied in any number of ways and all variations are encompassed by the present disclosure. This approach enables rapid detection and quantitation of TLR levels.

The antibodies as disclosed herein are also useful in *in situ* hybridization analysis such as of biopsy material. Such analysis enables the rapid diagnosis of levels of TLRs such as TLR-2 and TLR-4 according to the present invention.

Alternatively, the method for detection comprises detecting the level of expression in a cell of a polynucleotide encoding a TLR. Expression of such a polynucleotide may be determined using any suitable technique. For example, a labeled polynucleotide encoding a TLR may be utilized as a probe in a Northern blot of an RNA extract obtained from the cell. Preferably, a nucleic acid extract from the animal is utilized in concert with oligonucleotide primers corresponding to sense and antisense sequences of a polynucleotide encoding the kinase, or flanking sequences thereof, in a nucleic acid amplification reaction such as RT PCR. A variety of automated solid-phase detection techniques are also appropriate. For example, very large scale immobilized primer arrays (VLSIPS (trademark)) are used for the detection of nucleic acids as, for example, described by Fodor et al. (Science 251: 767-777, 1991) and Kazal et al. (Nature Medicine 2: 753-759, 1996). The above genetic techniques are well known to persons skilled in the art.

For example, for a TLR encoding RNA transcript, RNA is isolated from a cellular sample suspected of containing TLR RNA, e.g. total RNA isolated from human PBMCs. RNA can be isolated by methods known in the art, e.g. using TRIZOL (trademark) reagent (GIBCO-BRULife Technologies, Gaithersburg, Md.). Oligo-dT, or random-sequence oligonucleotides, as well as sequence-specific oligonucleotides can be employed as a primer in a reverse transcriptase reaction to prepare first-strand cDNAs from the isolated RNA. Resultant first-strand cDNAs are then amplified with sequence-specific oligonucleotides in PCR reactions to yield an amplified product.

"Polymerase chain reaction" or "PCR" refers to a procedure or technique in which amounts of a preselected fragment of nucleic acid, RNA and/or DNA, are amplified as described in U.S. Patent No. 4,683,195. Generally, sequence information from the ends of the region of interest or beyond is employed to design oligonucleotide primers. These primers will be identical or similar in sequence to opposite strands of the template to be amplified. PCR can be used to amplify specific RNA sequences and cDNA transcribed from total cellular RNA. See generally Mullis et al. (Quant. Biol. 51: 263, 1987; Erlich, eds., PCR Technology, Stockton Press, NY, 1989). Thus, amplification of specific nucleic acid sequences by PCR relies upon oligonucleotides or "primers" having conserved nucleotide sequences wherein the conserved sequences are deduced from alignments of related gene or protein sequences, e.g. a sequence comparison of mammalian TLR genes. For example, one primer is prepared which is predicted to anneal to the antisense strand and another primer prepared which is predicted to anneal to the sense strand of a cDNA molecule which encodes a TLR.

To detect the amplified product, the reaction mixture is typically subjected to agarose gel electrophoresis or other convenient separation technique and the relative presence of the TLR specific amplified DNA detected. For example, TLR amplified DNA may be detected using Southern hybridization with a specific oligonucleotide probe or comparing is electrophoretic mobility with DNA standards of known molecular weight. Isolation, purification and characterization of the amplified TLR DNA may be accomplished by excising or eluting the fragment from the gel (for example, see references Lawn et al:, Nucleic Acids Res. 2: 6103, 1981; Goeddel et al., Nucleic cids Res. 8: 4057-1980), cloning the amplified product into a cloning site of a suitable vector, such as the pCRII vector (Invitrogen), sequencing the cloned insert and comparing the DNA sequence to the known sequence of TLR. The relative amounts of TLR mRNA and cDNA can then be determined.

Real-time PCR is particularly useful in determining transcriptional levels of PCR genes. Determination of transcriptional activity also includes a measure of potential translational activity based on available mRNA transcripts. Real-time PCR as well as other PCR procedures use a number of chemistries for detection of PCR product including the binding of DNA binding fluorophores, the 5' endonuclease, adjacent liner and hairpin oligoprobes and the self-fluorescing amplicons. These chemistries and real-time PCR in general are discussed, for example, in Mackay et al., Nucleic Acids Res 30(6): 1292-1305, 2002; Walker, J. Biochem. Mol. Toxicology 15(3): 121-127, 2001; Lewis et al., J. Pathol. 195: 66-71, 2001.

The present description further discloses gene arrays and/or gene chips to screen for the up- or dowri-regulation of mRNA transcripts. This is particularly useful in identifying conditions which result in the up- or down-regulation of TLR gene transcripts. Furthermore, compounds can be readily screened which up- or down-regulate TLR transcripts and in particular TLR-2 and TLR-4 transcripts.

The present invention is further described by the following non-limiting Examples.

### EXAMPLE 1

### Effects of HCV on TLR-2 and TLR-4 expression

### Patients

The study group included 16 outpatients attending a specialist Liver Clinic at a university teaching hospital with biopsy proven chronic Hepatitis C (CHC) [Tables 3 and 4]. Thirty-two age- and sex-matched, asymptomatic volunteers with no history of liver disease, alcohol intake <20 g/day and normal liver function tests served as controls. Ethical approval was obtained from the South Eastern Area Health Service Research Ethics Committee, Department of Health, New South Wales, Australia.

### Blood Sampling

Peripheral blood was drawn using pyrogen-free needles, syringes and containers (Becton-Dickinson, Singapore). Plasma and serum were separated in a refrigerated centrifuge at 4°C and stored at -70°C in pyrogen-free polyethylene cryotubes (Nunc, Denmark) until analysis within six weeks of collection. Whole blood was used for determination of TLR expression on peripheral blood monocytes.

### Serum TNF-α Assay

Serum TNF-α was measured using the Quantikine HS Human TNF-γ Immunoassay (R&D Systems Inc., Minneapolis, USA), according to the manufacturer's instructions. Sensitivity of the assay was 0.5 pg/mL.

### Flow Cytometry for Determination of TLR Expression

Cell surface staining was performed on whole blood using the following anti-human monoclonal antibodies: anti-TLR-2-fluoroisothiocyanate (FITC) and anti-TLR-4-phycoerythrin (PE) (eBioscience) and anti-CD14-peridinin chlorophyll protein (PerCP) (Becton Dickinson, USA). Isotype matched non-binding controls were used for comparison. Ten thousand CD14-positive cells were acquired for each sample and dead cells were gated out based on their light scatter properties on FACSCaliber flow cytometer (Becton Dickinson, USA). At least two control patients were used for standardization purposes every flow cytometry session. TLR-2 and TLR-4 values were expressed as a ratio of the geometric mean fluorescence of individual study patients to mean control values for that session.

**TABLE 3**

| ***Patient Characterisitics*** | | |
|---|---|---|
| | **Patients** | **Controls** |
| Number of patients | 16 | 32 |
| Mean Age ± sd | 38.89 ± 8.67 | 35.44 ± 8.11 |
| Sex (M:F) | 10:6 | 20:12 |

**TABLE 4**

| ***Characteristics of the CHC patients*** | |
|---|---|
| **Characteristic** | |
| Genotype | 1: n-11, 2: n=2, 3: n=3 |
| Ishak histological activity score | Median: 4 range 2-7 |
| ALT | Median 109 range 55-350 U/L (N: 15-45 U/L) |

This Example demonstrates that CD14^{+ve} monocyte expression of TLR-2 and of TLR-4 is significantly up-regulated in CHC patients and that the TLR-2 level correlates significantly with circulating TNF-α ALT levels. These findings indicate that the up-regulation in the immune receptors may be a direct effect of the HCV. In addition cell signaling *via* TLR-2, but not TLR-4, likely contributes to the increased circulating levels of TNF-α and the corresponding increased ALT levels found in CHC. The good correlation between TNF-α and ALT levels may indicate a direct causative mechanism for some of the hepatic destruction in HCV infection.

### EXAMPLE 2

### Effect of HBV infection on TLR-2 expression

Eighteen non-cirrhotic patients with chronic Hepatitis B (CHB) and on-going viral replication (HBV DNA >200,000 genomes/mL, n=12 and 200-10,000 genomes/mL, n=6; Cobas Amplicor HBV Monitor (trademark) Test, USA) and 32 healthy control subjects were studied. TLR-2 and TLR-4 expression on CD14^{+ve} peripheral blood mononuclear cells (PBMC's) was measured by flow cytometry using anti-CD14 (Becton Dickinson) and anti-TLR-2 and anti-TLR-4 (eBioscience, USA) monoclonal antibodies. TLR expression was reassessed in five patients in whom HBV DNA fell from >200,000 to <200 genomes/mL following treatment with lamivudine. *In vitro* TLR-2 expression by PBMCs was measured in five control subjects at baseline and following stimulation for 20 h by partially purified recombinant HBV. TLR-2 expression (expressed as a ratio to control results) was significantly reduced in chronic hepatitis B patients with HBV DNA >200,000 genomes/mL (median: 0.63; range: 0.05-1.52) compared with controls (P=0.001) and those with HBV DNA 200-10,000 genomes/mL (median: 0.98; range: 0.94-1.17) (P=0.04). TLR-4 expression did not differ significantly between the three groups. TLR-2 expression normalised in each of the five lamivudine-treated CHB patients in whom HBV DNA became undetectable. *In vitro* expression of TLR-2 fell in a concentration-dependent manner following exposure to recombinant HBV.

This Example shows that HBV down-regulates expression of TLR-2 on PBMC's. It is proposed herein that the HBV-induced defect in innate immunity contributes to the development of persistent infection.

### EXAMPLE 3

### In vitro Analysis of HBV and TLR expression

### Hepatitis B Stimulation of Whole Blood

Peripheral blood was collected into Li-heparin tubes from 6 healthy volunteers. Whole blood was diluted with an equal volume of RPMI supplemented with antibiotics and 5% fetal bovine serum. 1 ml of diluted blood was stimulated with wildtype 1.5 (genotype A) HBV at a ratio of 1, 10 and 50 virus particles to 1 peripheral blood cell. Diluted blood was incubated at 37°C with gentle rotation in tightly capped 5ml polystyrene tubes. After 20 hours, culture supernatants were collected for TNF-alpha ELISA. The remaining cells were stained for flow cytometric analysis.

The HBV was prepared from transduction of cells using recombinant HBV/ Baculovirus system as previously described (Delaney 4th and Isom; Hepatology; 28:1134-46, 1998).

### TNF-alpha ELISA

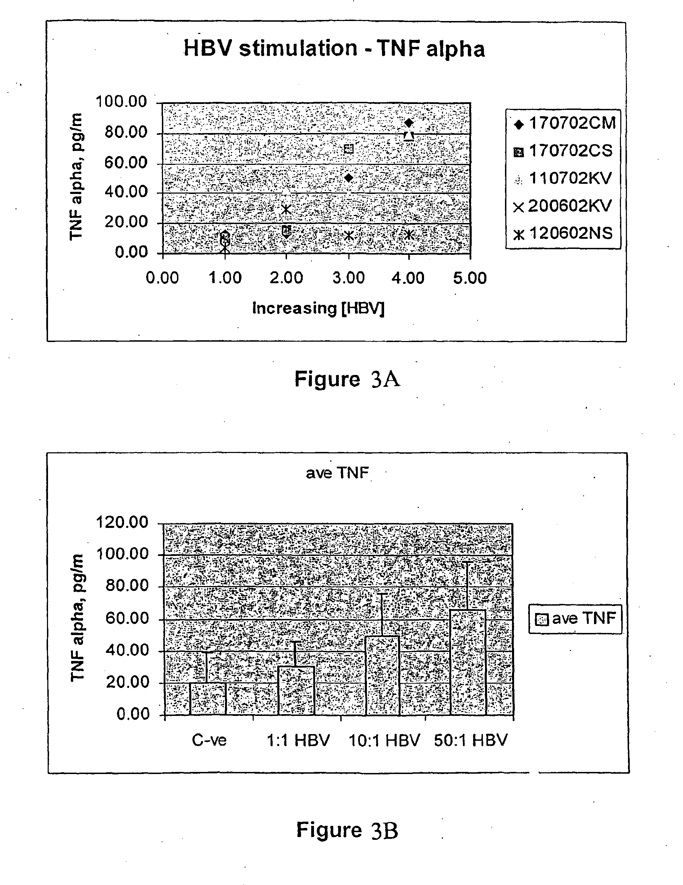

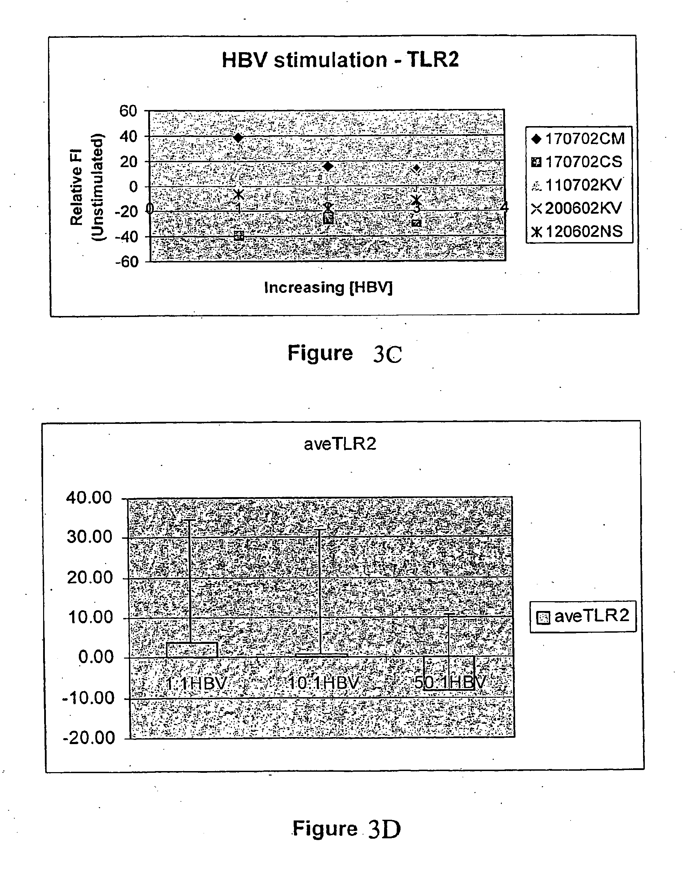

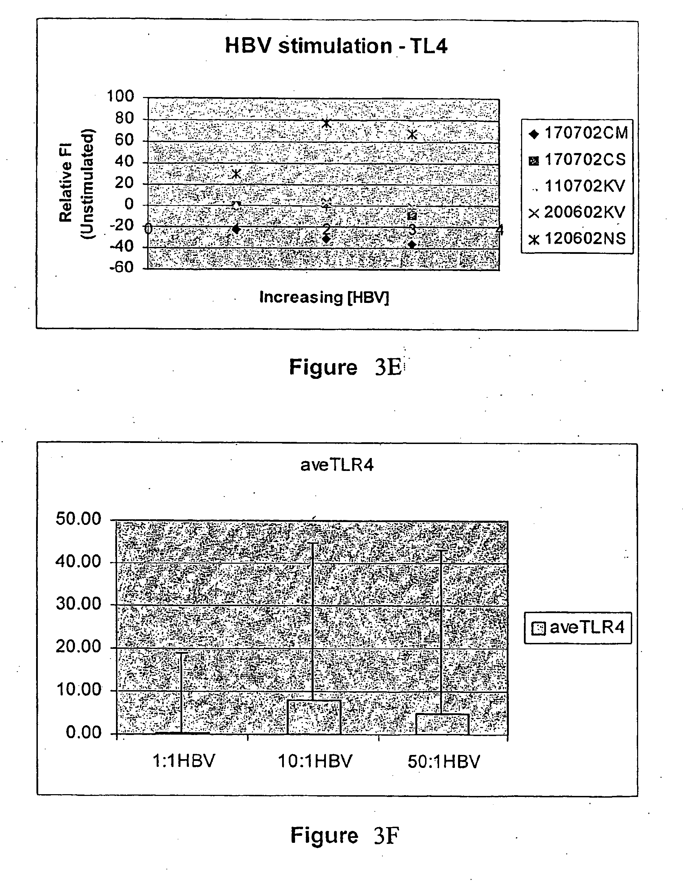

TNF-alpha from whole blood culture supernatants was measured by capture ELISA using TNF-alpha OptEIA set (BD) according to the manufacturer's specifications. Sensitivity was 8pg/ml. The TNF alpha results for all patients in shown in Figure 3 a and b.

### Flow Cytometry

Cell surface staining was performed on peripheral blood lymphocytes using CD14-PerCP (MφP9; BD), TLR2-FITC (TL2.1; eBioscience) and TLR4-PE (HTA125; eBioscience) antibodies. Appropriate isotype controls were used. Based on their scatter profile, monocytes were gated picking up the lymphocyte tail on a FACSCalibur flow cytometer (BD). A total of 8000 CD14 positive monocytes were acquired for each sample. Data was analysed using FlowJo software (Tree Star Inc.). Relative fluorescence intensity was determined by subtracting the geometric mean fluorescence intensity of the unstimulated control from the stimulated sample. The TLR2 and TLR4 results for all 6 patients following stimulation with wild type HBV is shown in Figure 3 (c, d, e, and f)

### HBV Baculovirus Infected HepG2 (Hershy)

The HBV was prepared from transduction of cells using recombinant HBV/ Baculovirus system as previously described (Delaney 4th and Isom; Hepatology. 1998, *supra*).

HepG2 (Hershy) cells were transdued with HBV 1:3 wildtype, or mock baculovirus infected and grown for 7 days prior to harvesting and staining for flow cytometry.
Some cells were reserved for total RNA extraction using the RNeasy mini kit (Qiagen) following the Manufacturer's specifications.

### Flow Cytometry

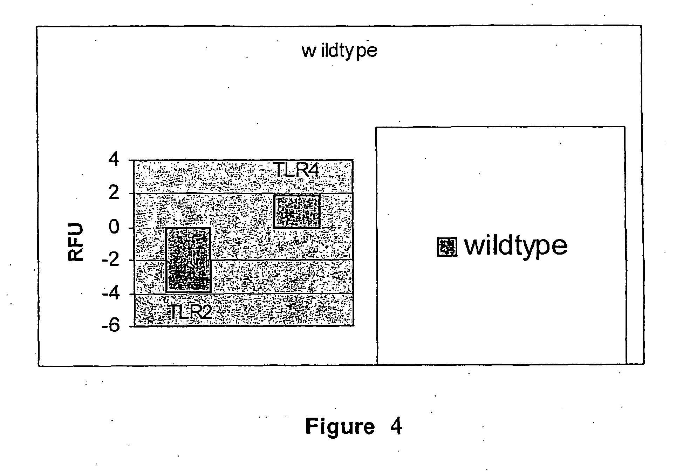

Cell surface staining was performed on HepG2 cells using TLR2-FITC (TL2.1; eBioscience) and TLR4-PE (HTA125; eBioscience) antibodies. Appropriate isotype controls were used. Dead cells were gated out based on their scatter profile, on a FACSCalibur flow cytometer (BD). A total of 10000 cells were acquired for each sample. Data was analysed using FlowJo software (Tree Star Inc.). Relative fluorescence intensity was determined by subtracting the geometric mean fluorescence intensity of the mock infected cells from the wildtype infected cells (Figure 4).

### QPCR

Total RNA was reversed transcribed using random hexamers prior to real time PCR analysis of the cDNA. PCR was performed in triplicate using TaqMan Universal PCR Master Mix and Assays-On-Demand Gene Expression Assay probes and primers (Applied Biosystems) in a final 10µl volume. Signal detection was via ABI Prism 7700 sequence detection system programmed to 50°C, 2 min; 95°C 10 min; 40 cycles of 95°C, 15 sec; 60°C, 1 min. The threshold cycle (*C*_{T}), values of each gene were compared with the *C*_{T} value of 18S (Δ*C*_{T}) and relative expression units (REU) calculated for each sample. Hence, REU= 2^*C*_{T}(gene of interest)-*C*_{T}(18S)= 2^Δ*C*_{T}.

### EXAMPLE 4

### Predicting outcome of therapeutic intervention

Using the methods provided in Example 2, subjects potentially exposed to HBV are screened for their levels of TLR-2 and/or TLR-4 prior to therapeutic intervention. When a subject exhibits a change in the level of TLR-2 and/or TLR-4 during early phase treatment (i.e. a trend to normalization of levels of TLR-2 and/or TLR-4) then this predicts that the therapy is working. The change in levels may be an elevation or reduction compared to pre-treatment and/or levels of standardized normal controls.

A similar diagnostic or predictive process may be applied to subjects potentially exposed or infected with HCV.

Those skilled in the art will appreciate that the invention described herein is susceptible to variations and modifications other than those specifically described. It is to be understood that the invention includes all such variations and modifications which fall within the claims' scope. The invention also includes all of the steps, features, compositions and compounds referred to or indicated in this specification, individually or collectively, and any and all combinations of any two or more of said steps or features, providing they fall within the claims' scope.

### BIBLIOGRAPHY

Adams et al., Cancer Res. 53: 4026-4034, 1993
Akashi et al., J Immunol. 164: 3471-3475, 2000
Akira et al., Nature Immunology 2: 675-680, 2001
Andus et al., Hepatology 13: 364-375, 1991
Baltimore, J. Virol. 54: 401-407, 1985
Bandyopadhyay and Temin, Mol. Cell. Biol. 4: 749-754, 1984
Berglund et al., Biotechnology 11: 916-920, 1993
Berkner et al., BioTechniques 6: 616-629, 1988
Berkner, Curr. Top. Microbiol. Immunol. 158: 39-66, 1992
Bieback et al., J Viral. 76: 8729-8736, 2002
Bigatello et al., Am J Gastroenterol. 82: 11-15, 1987
Breakefield and Geller, Mol. Neurobiol. 1: 339-371, 1987
Buchschacher and Panganiban, J. Virol. 66: 2731-2739, 1982\
Chothia et al., J. Mol. Biol. 196: 901, 1987
Chothia et al., J. Mol. Biol. 227: 799, 1992
Coligan et al., "Current Protocols in Immunology, John Wiley & Sons, Inc., 1991-1997
Cumber et al., J. Immunol. 149: 120-126, 1992
Davies & Riechmann, FEBS Lett. 339: 285-290, 1994
Delaney 4th and Isom; Hepatol., 28:1134-46, 1998.
Deviere et al., Gastroenterology 103: 1296-1301, 1992
Enomoto et al., Gastroenterology 115: 443-451, 1998
Enomoto et al., J Gastroenterol Hepatol. 15(Suppl): D20-D25, 2000
Erickson et al., Science 249: 527-533, 1990
Erlich, eds., PCR Technology, Stockton Press, NY, 1989
Fink et al., Ann. Rev. Neurosci. 19: 265-287, 1996
Fink et al., Hum. Gene Ther. 3: 11-19, 1992
Fodor et al., Science 251: 767-777, 1991
Freese et al., Biochem. Pharmacol. 40: 2189-2199, 1990
French J Biomed Sci. 8: 20-27, 2001
Fukui et al., J Hepatol. 12: 162-169, 1991
Gefter et al., Somatic Cell Genet. 3: 231-236, 1977
Genesca et al., Am J Gastroenterol. 94: 169-177, 1999
Glockshuber et al., Biochem. 29: 1363-1367, 1990
Goeddel et al., Nucleic Acids Res. 8: 4057-1980
Gorziglia and Kapikian, J. Virol. 66: 4407-4412, 1992
Haeberle et al., J Infect Dis. 186: 1199-1206, 2002
Hamers-Casterman et al., Nature 363: 446-448, 1993
Hanck et al., Gut 49: 106-111, 2001
Helseth et al., J. Virol. 64: 2416-2420, 1990
Hodgson, Bio/Technology 9: 19-21, 1991
Iimuro et al., Hepatology 26: 1530-1537, 1997
Johnson et al., "Peptide Turn Mimetics" in Biotechnology and Pharmacy, Pezzuto et al., Eds., Chapman and Hall, New York, 1993
Johnson et al., J. Virol. 66: 2952-2965, 1992
Jones et al., Nature 321: 522-525, 1986
Kabat et al., "Sequences of Proteins of Immunological Interest", U.S. Department of Health and Human Sciences, 1983
Kazal et al., Nature Medicine 2: 753-759, 1996
Kennet et al., Monoclonal Antibodies and Hybridomas: A New Dimension in Biological Analyses, pp 376-384, Plenum Press, New York, 1980
Khoruts et al., Hepatology 13: 267-276, 1991
Kohler and Milstein, Eur. J. Immunol. 6(7): 511-519, 1976
Köhler and Milstein, Nature 256: 495-499, 1975
Kostelny et al., J. Immunol. 148: 1547-1553, 1992
Kozbor et al., Methods in Enzymology 121: 140, 1986
Krebber et al., J. Immunol. Methods 201(1): 35-55,1997
Ku & Schutz, Proc. Natl. Acad. Sci. USA 92: 6552-6556, 1995
Kurt-Jones et al., Nat Immunol. 1: 398-401, 2000
Lawn et al., Nucleic Acids Res. 2: 6103, 1981
Lee et al., Scand J Gastroenterol. 31: 500-505, 1996
Lewis et al., J. Pathol. 195: 66-71, 2001
Lin et al., J Hepatol. 22: 165-172, 1995
Chan et al., Scand J Gastroenterol. 32: 942-946, 1997
Liu et al., Proc. Natl. Acad. Sci. USA 84: 3439-3443, 1987
Mackay et al., Nucleic Acids Res 30(6): 1292-1305, 2002
Madzak et al., J. Gen. Virol. 73: 1533-1536, 1992
Manrekar et al., Alcohol Clin Exp Res. 21: 1226-1231, 1997
Margolskee, Curr. Top., Microbiol. Immunol. 158: 67-95, 1992;
Matsumoto et al., Biochem Biophys Res Commun. 293: 1364-1369, 2002
Medzhitov et al., N Engl J Med. 343: 338-344, 2000
Miller et al., J. Virol. 62: 4337-4345, 1988
Miller et al., Mol. Cell. Biol. 5: 431-437, 1985
Miller, Curr. Top. Microbiol, Immunol. 158: 1-24, 1992
Moss, Curr. Top. Microbiol, Immunol. 158: 25-38, 1992
Moss, Proc. Natl. Acad. Sci. USA 93: 11341-11348, 1996
Mullis et al., Quant. Biol. 51: 263, 1987
Muzyczka, Curr. Top. Microbiol. Immunol. 158: 97-129, 1992;
Naldini et al., Science 272: 263-267, 1996
Nanji et al., Am J Pathol. 142: 367-373, 1993
Neuman et al., J Gastroenterol Hepatol. 17: 196-202, 2002
O'Reilly et al., Biotechniques 25: 824-830, 1998
Ohi et al., Gene 89: 279-282, 1990;
Page et al., J. Virol. 64: 5270-5276, 1990
Petropoulos et al., J. Viol. 66: 3391-3397, 1992
Plückthun et al., In Antibody engineering. A practical approach, 203-252, 1996
Plünckthun, Biochem 31: 1579-1584, 1992
Pugh et al., Br J Surg. 60: 646-649, 1973
Quantin et al., Proc. Natl. Acad. Sci. USA 89: 2581-2584, 1992
Reiter et al., Biochem. 33: 5451-5459, 1994
Reiter et al., Cancer Res. 54: 2714-2718, 1994
Reiter et al., J. Biol. Chem. 269: 18327-18331, 1994
Remington's Pharmaceutical Sciences, 18th Ed., 1990, Mack Publishing, Company, Easton, PA, U.S.A.
Richmann et al., Nature 332: 323-327, 1988
Rivera et al., Am JPhysiol. 275: G1252-1258, 1998
Rosenfeld et al., Cell 68: 143-155, 1992
Russell and Hirata, Nature Genetics 18: 323-328, 1998
Schafer et al., Z Gastroenterol. 33: 503-508, 1995
Schneider et al., Nature Genetics 18: 180-183, 1998
Shimada et al., J. Clin. Invest. 88: 1043-1047, 1991
Shulman et al., Nature 276: 269-270, 1978
Sorge et al., Mol. Cell. Biol. 4: 1730-1737, 1984
Stratford-Perricaudet et al., Hum. Gene Ther. 1: 241-256, 1990
Summerton and Weller, Antisense and Nucleic Acid Drug Development 7: 187-195, 1997
Takeuchi et al., Immunity 11: 443-451, 1999
Tapping et al., J Immunol. 165: 5780-5787, 2000
Tilg et al., Gastroenterology. 103: 264-274, 1992
Tilg et al., Hepatology 18: 1132-1138, 1993
Toyama et al., Monoclonal Antibody, Experiment Manual", published by Kodansha Scientific, 1987
Trowbridge, J. Exp. Med. 148(1): 313-323, 1978
van Leeuwen et al., Surgery 110: 169-174, 1991
Verhoeyen et al., Science 239: 1534-1536, 1988
Volk et al., J. Virol. 42(1): 220-227, 1982
von Baehr et al., Gut 47: 281-287, 2000
Walker, J Biochem. Mol. Toxicology 15(3): 121-127, 2001
Ward et al., Nature 341: 544-546, 1989
Webber et al., Mol. Immunol. 32: 249-258, 1995
Wells, Methods Enzymol. 202: 2699-2705, 1991
Wilkinson et al., Nucleic Acids Res. 20: 2233-2239, 1992
Winter and Milstein, Nature 349: 293, 1991
Yin et al., Gastroenterology 117: 942-952, 1999
Yoshimura et al., J Immunol. 163: 1-5, 1999

## Claims

1. An *in vitro* diagnostic method for determining whether a subject will respond to an anti-HCV treatment or an anti-HBV treatment said method comprising determining the level of TLR-2 and/or TLR-4 wherein the potential efficacy of the treatment is determined by an elevated level of TLR-2 and TLR-4 in a subject with HCV and a reduced level of TLR-2 in a subject with HBV.

2. An *in vitro* diagnostic method for monitoring a response to treatment of a subject against HCV or HBV infection said method comprising determining the level of TLR-2 and/or TLR-4 wherein the efficacy of the anti-HBV treatment is determined by an elevation in the level of TLR-2 and the efficacy of the anti-HCV treatment is determined by a reduction in the level of TLR-2 and TLR-4 compared to pre-treatment levels and/or standardized controls.

3. An *in vitro* diagnostic method for predicting the outcome of a therapeutic protocol directed against infection by HCV or HBV said method comprising determining the level of TLR-2 and/or TLR-4 wherein the efficacy of the therapeutic protocol is determined by an elevation in the level of TLR-2 during anti-HBV treatment and by a reduction in the level of TLR-2 and TLR-4 during anti-HCV treatment compared to pre-treatment levels and/or standardized controls.

4. The method of any one of claims 1 to 3 wherein the level of TLR-2 or TLR-4 is determined by the amount of mRNA encoding same, or at the protein level.

5. The use of an agent which up-regulates or down-regulates the level of TLR-2 and/or TLR-4 in the manufacture of a medicament for treating a subject infected with HCV or HBV wherein the agent down-regulates TLR-2 and TLR-4 in anti-HCV treatment and up-regulates TLR-2 in anti-HBV treatment, wherein said agent is selected from an antibody specific for TLR-2 or TLR-4 or an antigen-binding fragment thereof, a sense or antisense nucleic acid molecule to TLR-2 or TLR-4 and RNAi, siRNA, a ribozyme or a DNAzyme to TLR-2 or TLR-4.

6. The method of any one of claims 1 to 4 or use of claim 5 wherein the subject is selected from a human, non-human primate, livestock animal, companion animal, or avian species.

7. The use of claim 5 or 6 wherein the antibody is a monoclonal and/or a deimmunized antibody.

8. An agent which up-regulates or down-regulates the level of TLR-2 and/or TLR-4 for use in treating a subject infected with HCV or HBV wherein the agent down-regulates TLR-2 and TLR-4 in anti-HCV treatment and up-regulates TLR-2 in anti-HBV treatment, wherein said agent is selected from an antibody specific for TLR-2 or TLR-4 or an antigen-binding fragment thereof, a sense or antisense nucleic acid molecule to TLR-2 or TLR-4 and RNAi, siRNA, a ribozyme or a DNAzyme to TLR-2 or TLR-4.

9. An agent for a treatment as defined in claim 8 wherein said agent is as defined in claim 7 and/or said subject is as defined in claim 6.

## Patentansprüche

1. In vitro-Diagnoseverfahren zum Bestimmen ob eine Testperson auf eine anti-HCV-Behandlung oder eine anti-HBV-Behandlung anspricht, wobei das Verfahren das Bestimmen des TLR-2- und/oder TLR-4-Gehalts umfasst, worin die potentielle Wirksamkeit der Behandlung durch einen erhöhten Gehalt von TLR-2 und TLR-4 in einer Testperson mit HCV und einen verringerten Gehalt von TLR-2 in einer Testperson mit HBV bestimmt wird.

2. In vitro-Diagnoseverfahren zur Überwachung eines Ansprechens einer Testperson auf eine Behandlung gegen eine HCV- oder HBV-Infektion, umfassend das Bestimmen des TLR-2- und/oder TLR-4-Gehalts, worin die Wirksamkeit der anti-HBV-Behandlung durch eine Erhöhung des Gehalts von TLR-2 bestimmt wird und die Wirksamkeit der anti-HCV-Behandlung durch eine Verringerung des Gehalts von TLR-2 und TLR-4 im Vergleich mit Gehalten vor der Behandlung und/oder standardisierten Kontrollen bestimmt wird.

3. In vitro-Diagnoseverfahren zum Vorhersagen des Ergebnisses eines therapeutischen Protokolls, das gegen eine Infektion durch HCV oder HBV gerichtet ist, wobei das Verfahren das Bestimmen des TLR-2-und/oder TLR-4-Gehalts umfasst, worin die Wirksamkeit des therapeutischen Protokolls durch eine Erhöhung des Gehalts von TLR-2 während einer anti-HBV-Behandlung und durch eine Verringerung des Gehalts von TLR-2 und TLR-4 während einer anti-HCV-Behandlung im Vergleich mit Gehalten vor der Behandlung und/oder standardisierten Kontrollen bestimmt wird.

4. Verfahren nach einem der Ansprüche 1 bis 3, worin der Gehalt von TLR-2 oder TLR-4 durch die Menge m-RNA, die für dieselben codiert, oder auf dem Proteinniveau bestimmt wird.

5. Verwendung eines Mittels, das den Gehalt von TLR-2 und/oder TLR-4 hoch- oder hinunterreguliert, bei der Erstellung eines Medikaments zur Behandlung einer Testperson, die mit HCV oder HBV infiziert ist, worin das Mittel TLR-2 und TLR-4 in einer anti-HCV-Behandlung hinunterreguliert und TLR-2 in einer anti-HBV-Behandlung hochreguliert, worin das Mittel ausgewählt wird aus einem Antikörper, der spezifisch ist für TLR-2 oder TLR-4 oder ein Antigen-Bindungsfragment davon, einem sense- oder antisense-Nukleinsäuremolekül für TLR-2 oder TLR-4 und RNAi, siRNA, einem Ribozym oder einem DNAzym für TLR-2 oder TLR-4.

6. Verfahren nach einem der Ansprüche 1 bis 4 oder Verwendung nach Anspruch 5, worin die Testperson ausgewählt wird aus einem Menschen, nicht-humanen Primaten, Vieh, Haustier und Vogelarten.

7. Verwendung nach Anspruch 5 oder 6, worin der Antikörper ein monoklonaler und/oder deimmunisierter Antikörper ist.

8. Mittel, das den Gehalt von TLR-2 und/oder TLR-4 hoch- oder hinunterreguliert, zur Verwendung bei der Behandlung einer Testperson, die mit HCV oder HBV infiziert ist, worin des Mittel TLR-2 und TLR-4 bei einer anti-HCV-Behandlung hinunterreguliert und TLR-2 in einer anti-HBV-Behandlung hochreguliert, worin das Mittel ausgewählt ist aus einem Antikörper, der spezifisch ist für TLR-2 oder TLR-4 oder ein Antigen-Bindungsfragment davon, einem sense- oder antisense-Nukleinsäuremolekül für TLR-2 oder TLR-4 und RNAi, siRNA, einem Ribozym oder einem DNAzym für TLR-2 oder TLR-4.

9. Mittel für eine Behandlung nach Anspruch 8, worin das Mittel wie in Anspruch 7 definiert ist und/oder die Testperson wie in Anspruch 6 definiert ist.

## Revendications

1. Procédé de diagnostic *in vitro* destiné à déterminer si un sujet réagira à un traitement anti-VHC ou à un traitement anti-VHB, ledit procédé comprenant une étape consistant à déterminer le niveau de TLR-2 et / ou de TLR-4, dans lequel l'efficacité potentielle du traitement est déterminée par un niveau élevé de TLR-2 et de TLR-4 chez un sujet porteur du VHC et un niveau réduit de TLR-2 chez un sujet porteur du VHB.

2. Procédé de diagnostic *in vitro* destiné à surveiller une réponse au traitement d'un sujet contre une infection par le VHC ou le VHB, ledit procédé comprenant une étape consistant à déterminer le niveau de TLR-2 et/ou de TLR-4, dans lequel l'efficacité du traitement anti-VHB est déterminée par une augmentation du niveau de TLR-2 et l'efficacité du traitement anti-VHC est déterminée par une réduction du niveau de TLR-2 et de TLR-4 par rapport à des niveaux de traitements préalables et/ou à des contrôles normalisés.

3. Procédé de diagnostic *in vitro* destiné à prévoir les résultats d'un protocole thérapeutique dirigé contre une infection par le VHC ou le VHB, ledit procédé comprenant une étape consistant à déterminer le niveau de TLR-2 et/ou de TLR-4, dans lequel l'efficacité du protocole thérapeutique est déterminée par une augmentation du niveau de TLR-2 au cours d'un traitement anti-VHB et par une réduction du niveau de TLR-2 et de TLR-4 au cours d'un traitement anti-VHC par rapport à des niveaux de traitements préalables et/ou à des contrôles normalisés.

4. Procédé selon l'une quelconque des revendications 1 à 3, dans lequel le niveau de TLR-2 ou de TLR-4 est déterminé par la quantité d'ARNₘ codant pour celui-ci, ou au niveau du taux de protéine.

5. Utilisation d'un agent qui régule à la hausse ou à la baisse le niveau de TLR-2 et/ou de TLR-4 dans la fabrication d'un médicament pour traiter un sujet infecté avec le VHC ou le VHB, dans lequel l'agent régule à la baisse le TLR-2 et le TLR-4 dans le traitement anti-VHC et régule à la hausse le TLR-2 dans le traitement anti-VHB, dans lequel ledit agent est sélectionné dans le groupe constitué par un anticorps spécifique du TLR-2 ou du TLR-4 ou un fragment à liaison antigène de celui-ci, un molécule d'acide nucléique sens ou antisens à TLR-2 ou à TLR-4 et ARN interférent, si RNA, une ribozyme ou une DNAzyme à TLR-2 ou à TLR-4.

6. Procédé selon l'une quelconque des revendications 1 à 4 ou utilisation selon la revendication 5, dans lequel le sujet est sélectionné dans le groupe constitué par les êtres humains, des primates non humains, des têtes de bétail, des animaux de compagnie, ou des espèce aviaires.

7. Utilisation selon la revendication 5 ou la revendication 6, dans laquelle l'anticorps est un anticorps monoclonal et / ou désimmunisé.

8. Agent qui régule à la hausse ou à la baisse le niveau de TLR-2 et/ou de TLR-4, destiné à être utilisé pour traiter un sujet infecté avec le VHC ou le VHB, dans lequel l'agent régule à la baisse le TLR-2 et le TLR-4 dans le traitement anti-VHC, et régule à la hausse le TLR-2 dans le traitement anti-VHB, dans lequel ledit agent est sélectionné dans le groupe constitué par un anticorps spécifique du TLR-2 ou du TL-4 ou un fragment à liaison antigène de celui-ci, un molécule d'acide nucléique sens ou antisens à TLR-2 ou à TLR-4 et ARN interférent, si RNA, une ribozyme ou une DNAzyme à TL-2 ou à TLR-4.

9. Agent destiné à un traitement selon la revendication 8, dans lequel ledit agent est selon la revendication 7 et/ou ledit sujet est selon la revendication 6.
